# EUROPEAN PATENT SPECIFICATION

(11) **EP 4 276 183 B1**
(45) Date of publication and mention of the grant of the patent: **02.12.2026**
(21) Application number: 23186136.0
(22) Date of filing: 06.11.2018
(51) Int. Cl.: A61K 31/7105, A61K 31/7088, A61K 45/06, A61K 47/42, A61K 47/46, A61P 35/00, A61P 43/00, C12N 15/113

(54) **PHARMACEUTICAL COMPOSITION COMPRISING MIRNA-657 FOR USE IN TREATING CANCER**
PHARMAZEUTISCHE ZUSAMMENSETZUNG ENTHALTEND MIRNA-657 ZUR BEHANDLUNG VON KREBS
COMPOSITION PHARMACEUTIQUE COMPRENANT DU MIRNA-657 POUR LE TRAITEMENT DU CANCER

(30) Priority: 09.11.2017 JP 2017216336
(43) Date of publication of application: 15.11.2023
(62) Document number(s) of the earlier application(s) in accordance with Art. 76 EPC: 18876388.2 / 3 708 173
(73) Proprietor: Purmx Therapeutics, Inc., Minami-ku Hiroshima-shi Hiroshima 734-8553 (JP)
(72) Inventor: TAHARA, Hidetoshi, Hiroshima, 734-8553 (JP); KINEHARA, Masaki, Hiroshima, 734-8553 (JP); YAMAMOTO, Yuki, Hiroshima, 734-8553 (JP)
(74) Representative: Weickmann & Weickmann PartmbB

(56) References cited:
- WO-A1-2012/108843
- CA-A1- 2 977 624
- XI HAO ET AL: "hsa-miR-631 resensitizes bortezomib-resistant multiple myeloma cell lines by inhibiting UbcH10", ONCOLOGY REPORTS, vol. 37, no. 2, 14 December 2016 (2016-12-14), pages 961 - 968, XP055879318, ISSN: 1021-335X, DOI: 10.3892/or.2016.5318
- FU DEWANG ET AL: "MiR-631/ZAP70: A novel axis in the migration and invasion of prostate cancer cells", BIOCHEMICAL AND BIOPHYSICAL RESEARCH COMMUNICATIONS, vol. 469, no. 3, 1 January 2016 (2016-01-01), Amsterdam NL, pages 345 - 351, XP055880035, ISSN: 0006-291X, DOI: 10.1016/j.bbrc.2015.11.093
- LISHENG ZHANG ET AL: "MicroRNA-657 promotes tumorigenesis in hepatocellular carcinoma by targeting transducin-like enhancer protein 1 through nuclear factor kappa B pathways", HEPATOLOGY, vol. 57, no. 5, 5 April 2013 (2013-04-05), US, pages 1919 - 1930, XP055227066, ISSN: 0270-9139, DOI: 10.1002/hep.26162

## Description

### Technical Field

The present invention relates to a pharmaceutical composition for cancer therapy comprising a transcription or processing product of a gene encoding a particular miRNA.

### Background Art

MicroRNA (hereinafter miRNA) is a functional nucleic acid that is encoded on the genome and ultimately becomes a minuscule RNA of about 20 - 25 bases long through a multistep production process. miRNA is classified as a functional ncRNA (non-coding RNA), and it is being elucidated that it plays an important role in various biological phenomena (such as regulation of gene expression etc.). Various miRNAs that has come to be well-known thus far including human miRNA are registered in the miRBase (see http://www.mirbase.org/).

miRNA is indicated to be associated with the onset and progression of e.g. cancer, cardiovascular disease, neurodegenerative disease, psychiatric disease, chronic inflammatory disease, and the like. Particularly in recent years, it has been indicated that miRNA is deeply involved in canceration or aging of cells.

For example, Patent Literature 1 describes that miR-22 promotes the aging of cells and suppresses invasion and metastasis of cancer. Moreover, Patent Literature 2 describes that a composition comprising miR-34 may be employed for cancer therapy. Patent Literature 3 discloses a pharmaceutical composition for the treatment of cancer, wherein the composition comprises an miRNA as an active ingredient. Example 1 discloses the construction of an miRNA screening library. The list of miRNAs is extremely long and it also includes miR-657. However, this particular miRNA is not further examined and there is no support that it can actually be used for successfully treating cancer. Non-patent Literature 1 discloses an miR-657 precursor that provides tumorigenesis in hepatocellular cancer.

### Citation List

[Patent Literature 1] WO2011/078037
[Patent Literature 2] WO2008/137867
[Patent Literature 3] CA 2 977 624
[Non-patent Literature 1] Lisheng Zhang et al., Hepatology, 2013, vol.57, no.5, pp. 1919-1930

### Summary of the Invention

### Problems to be Solved by the Invention

The present inventors diligently searched for miRNAs having therapeutic effect against cancer from among a great number of miRNAs.

### Means for Solving the Problems

As a result, the present inventors found particular miRNAs that have significantly stronger cancer therapeutic effect than the miRNAs described in prior art, thus arriving at the completion of the present invention.

The present invention is defined by the appended claims. In other words, the present invention relates to a pharmaceutical composition for cancer therapy comprising a transcription or processing product of a gene encoding a miRNA, characterized in that said miRNA is miR-657. Other miRNAs, in particular miR-3140, miR-137, and miR-631 are described for comparative purposes only.

One embodiment of the present invention is characterized in that said cancer is a solid cancer.

One embodiment of the present invention is characterized in that said solid cancer is colon cancer, pancreatic cancer, tongue cancer, mesothelioma, uterine sarcoma, osteosarcoma, breast cancer, lung cancer, or head and neck cancer.

One embodiment of the present invention is characterized in that said transcription or processing product of a gene encoding a miRNA is a pri-miRNA, a pre-miRNA, a double-stranded mature-miRNA, a single-strand mature-miRNA expressed from the 5'-end of a pre-miRNA, or a single-strand mature-miRNA expressed from the 3'-end of a pre-miRNA.

The present invention is characterized in that said miRNA is:
(i) a mature-miRNA consisting of a sequence represented by SEQ ID NO. 11;
(ii) a miRNA having substitution, addition, and/or deletion of 1 - 5 bases to a mature-miRNA consisting of a sequence represented by SEQ ID NO. 11, as well as having cancer therapeutic effect; or
(iii) a miRNA having 80% or more sequence homology against a mature-miRNA consisting of a sequence represented by SEQ ID NO. 11, as well as having cancer therapeutic effect.

One embodiment of the present invention is characterized in that said miRNA is chemically modified.

One embodiment of the present invention is characterized in that said chemical modification is one or more chemical modifications selected from the group consisting of LNA-tion, BNA-tion, ENA-ation, 2'-OMe modification, phosphorothioation, S-TuD-ation, morpholino modification, peptide addition, glycosylation, aptamer addition, hydrophobic molecule addition, polymer addition, and addition of unmodified DNA.

One embodiment of the present invention is characterized in that said pharmaceutical composition further comprises a nucleic acid transfection agent.

One embodiment of the present invention is characterized in that said transfection agent is a lipid-based transfection agent, a polymer-based transfection agent, a magnetic particle-based transfection agent, an exosome for nucleic acid delivery, or a viral protein for nucleic acid delivery.

One embodiment of the present invention is characterized in that said transfection agent is a transfection agent comprising a peptide represented by amino acid sequences GGGGDD (G4D2), GGGGGGDD (G6D2), GGGGGGGGDD (G8D2), GGGGGGGGGGDD (G10D2), AAAAAAD (A6D), AAAAAADD (A6D2), AAAAAAK (A6K), AAAAAAKK (A6K2), VVVVVVD (V6D), VVVVVVDD (V6D2), VVVVVVK (V6K), VVVVVVKK (V6K2), LLLLLLD (L6D), LLLLLLDD (L6D2), LLLLLLK (L6K), or LLLLLLKK (L6K2).

One embodiment of the present invention is characterized in that the pharmaceutical composition of the present invention is for topical administration.

One embodiment of the present invention is characterized in that the pharmaceutical composition of the present invention is used in combination with other anticancer agents.

One embodiment of the present invention is characterized in that said other anticancer agents are one or more anticancer agents selected from the group consisting of an alkylating agent, a platinum preparation, a metabolism antagonist, a topoisomerase inhibitor, a microtubular inhibitor, an anti-cancerous antibiotic, a molecular target drug, a hormone preparation, an immunomodulation drug, an interferon, an interleukin, and a plant-derived anticancer agent.

### Brief Description of the Drawings

Figure 1 describes the flow of miRNA screening in the Examples.
Figure 2 describes the flow of miRNA screening in the Examples.
Figure 3 shows four miRNAs that show growth inhibition effect against numerous types of cancer cells sorted in miRNA screening.
Figure 4 shows the effect of the miRNA of the present invention (miR-657) and comparative miRNAs on tongue cancer cell strains (HSC-4 cells and OSC19 cells).
Figure 5 shows the effect of the miRNA of the present invention (miR-657) and comparative miRNAs on tongue cancer cell strain (SAS cells).
Figure 6 shows the effect of comparative miRNAs on mesothelioma cell strain (MSTO-211H cells).
Figure 7 shows the effect of comparative miRNAs on mesothelioma cell strain (EHMES-10 cells).
Figure 8 shows the effect of comparative miRNAs on uterine sarcoma cell strain (MES-SA cell).
Figure 9 shows the effect of comparative miRNAs on osteosarcoma cell strain (U2-OS cells).
Figure 10 shows the effect of comparative miRNAs on cancer stem cell strains (MDA-MB231-luc-D3H2LN cells).
Figure 11 shows the effect of comparative miRNAs on lung cancer cell strain (A549 cells).
Figure 12 shows the effect of comparative miRNAs on large intestine cancer cell strains (SW620 cells, SW480 cells, and HT29 cells).
Figure 13 shows the cancer therapeutic effect of comparative miRNAs *in vivo.*
Figure 14 shows imaging analysis results of tumor tissues at 12, 19, 26, and 33 days after tumor cell transplantation.
Figure 15 shows a graph quantifying the imaging analysis results from Figure 14.
Figure 16 shows the results of comparing tumor weights at endpoint.
Figure 17 shows imaging analysis results of tumor tissues after tumor cell transplantation.
Figure 18 shows the results of comparing tumor weights at endpoint.
Figure 19 shows the schedule in Experiment 7.
Figure 20 shows the imaging results when miR-3140-3p (a comparative miRNA) is administered once.
Figure 21 shows the imaging results when miR-3140-3p (a comparative miRNA) is administered once.
Figure 22 shows survival rates of mice when miR-3140-3p (a comparative miRNA )is administered once.
Figure 23 shows the imaging results when miR-3140-3p (a comparative miRNA) is administered twice.
Figure 24 shows the imaging results when miR-3140-3p (a comparative miRNA) is administered twice.
Figure 25 shows the survival rates of mice when miR-3140-3p (a comparative miRNA) is administered twice.
Figure 26 shows the Imaging results when miR-3140-3p (a comparative miRNA) is administered three times.
Figure 27 shows the Imaging results when miR-3140-3p (a comparative miRNA) is administered three times.
Figure 28 shows the survival rates of mice when miR-3140-3p (a comparative miRNA) is administered three times.
Figure 29 shows the results of Experiment 8.
Figure 30 shows the comparison of tumor suppression effect between miR-3140-3p (a comparative miRNA) and cisplatin.
Figure 31 shows the comparison of tumor suppression effect between miR-3140-3p (a comparative miRNA) and cisplatin.
Figure 32 shows the comparison of tumor suppression effect between miR-3140-3p (a comparative miRNA) and cisplatin.
Figure 33 shows the comparison of survival rates between miR-3140-3p (a comparative miRNA) administration group and cisplatin administration group.

### Description of Embodiments

The present invention relates to a pharmaceutical composition for cancer therapy comprising a transcription or processing product of a gene encoding a miRNA (micro-RNA) miR-657 as defined in claim 1. A general miRNA is biosynthesized via a continuous process. The primary transcription product of a gene encoding a miRNA is called a Primary miRNA transcript (pri-miRNA), and generally has a stem-loop hairpin structure. Pri-miRNA is cleaved by a microprocessor complex, takes a hairpin form by Drosha which is a RNase III-series enzyme, and precursor miRNA (pre-miRNA) which is an intermediate precursor of about 70 bases is produced. The pre-miRNA is then transported from the nucleus to the cytoplasm. In the cytoplasm, it is further cleaved by Dicer which is another RNase III enzyme, and a double-stranded mature miRNA is produced. In general, among the two strands, "-5p" is added to that expressed from the 5'-end of the precursor and "-3p" is added to that expressed from the 3'-end, and are represented as "hsa-miR-21-5p" and "hsa-miR-21-3p". Note that in principle, well-known miRNAs are registered in the miRBase (http://www.mirbase.org/).

Note that only one of the strands in the mature miRNA may exert the desired effect, or each of the stands may exert the desired effect, or the desired effect may be exerted in the double-stranded state. Moreover, the desired effect may also be exerted in pri-miRNA state or pre-miRNA state.

The nucleic acid comprised in the composition of the present invention is a transcription or processing product of a gene encoding miR-657, and may also be a variant or a modification that retains the function of the aforementioned nucleic acid as defined in claim 1.

The sequence of the transcription or processing product of the gene encoding miR-3140 used in one comparative example is as follows.

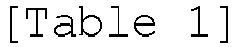

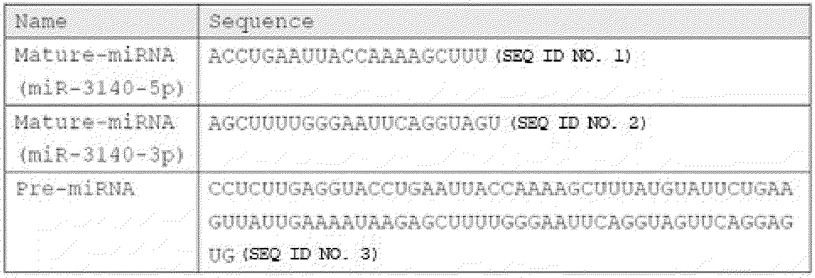

Note that the sequence of the intron region encoding the pri-miRNA of miR-3140 is shown in SEQ ID NO. 4.

The sequence of the transcription or processing product of the gene encoding miR-137 used in one comparative example is as follows.

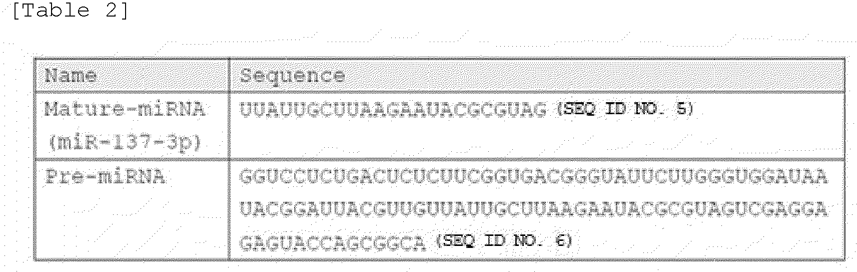

Note that the sequence of the intron region encoding the pri-miRNA of miR-137 is shown in SEQ ID NO. 7.

The sequence of the transcription or processing product of the gene encoding miR-631 used in one comparative example is as follows.

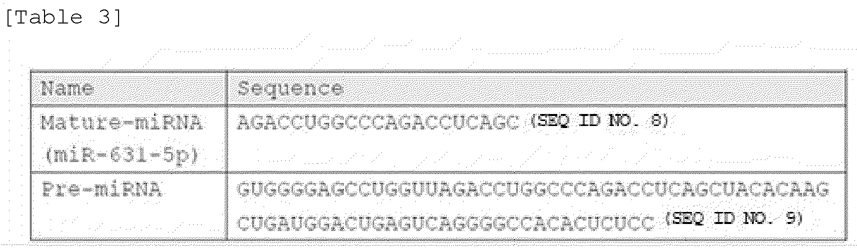

Note that the sequence of the intron region encoding the pri-miRNA of miR-631 is shown in SEQ ID NO. 10.

The sequence of the transcription or processing product of the gene encoding miR-657 used according to the present invention is as follows.

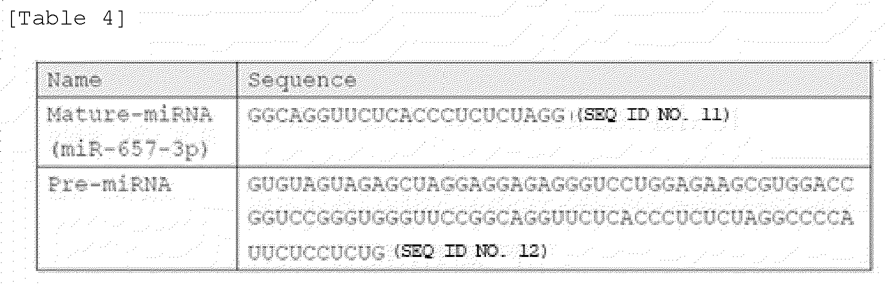

Note that the sequence of the intron region encoding the pri-miRNA of miR-657 is shown in SEQ ID NO. 13.

The nucleic acid comprised in the composition of the present invention may be e.g. a nucleic acid having substitution, addition, and/or deletion of 1, 2, 3, 4, or 5 bases to a mature-miRNA consisting of a sequence represented by SEQ ID NO. 11, as well as having the effect of inhibiting cancer cell growth. The substitution of a base to the mature-miRNA may be e.g. a conservative substitution of RNA known in the art.

Moreover, the nucleic acid comprised in the composition of the present invention may be e.g. a nucleic acid having 80% or more (preferably, 85% or more, 90% or more, 95% or more) sequence homology (or sequence identity) to a mature-miRNA consisting of a sequence represented by SEQ ID NO. 11, as well as having the effect of inhibiting cancer cell growth.

A mature-miRNA consisting of a sequence represented by SEQ ID NO. 11 can be easily manufactured by a RNA synthesis equipment commonly used in the art, and a nucleic acid having a particular base substituted, added, and/or deleted can similarly be easily manufactured. Moreover, numerous companies accept commissioned synthesis of nucleic acids, and it is also easy to obtain RNA of the desired sequence from such companies. Accordingly, those skilled in the art will be able to investigate the nature and function of a variant or modification of a mature-miRNA consisting of a sequence represented by SEQ ID NO. 11 by conventional means without excessive burden.

Moreover, the nucleic acid comprised in the composition of the present invention may have received a chemical modification well-known in the art with the purpose of improving the stability or specificity etc. of RNA. Chemical modification that may be employed in the present invention may be e.g. LNA (Locked Nucleic Acid)-tion, BNA (Bridged Nucleic Acid)-tion, ENA (2'-O,4'-C-Ethylene-bridged Nucleic Acids)-tion, 2'-OMe modification, phosphorothioation (S-ation), S-TuD (Synthetic Tough Decoy)-ation, morpholino modification, peptide addition, glycosylation, aptamer addition, hydrophobic molecule addition such as cholesterol, polymer addition such as PEG, or addition of unmodified DNA. These chemical modifications can be performed by a well-known means known in the art.

The present invention can be employed for various cancer therapies, e.g. it can be favorably employed for solid cancer. More preferably, the subject of the present invention may be colon cancer, pancreatic cancer, tongue cancer, mesothelioma, uterine sarcoma, osteosarcoma, breast cancer, lung cancer, or head and neck cancer.

In order to appropriately introduce the present invention to cancer cells, the composition of the present invention may further comprise a nucleic acid transfection agent. Examples of the nucleic acid transfection agent that may be employed for the present invention include a lipid-based transfection agent, a polymer-based transfection agent, a magnetic particle-based transfection agent, an exosome for nucleic acid delivery, or a viral protein for nucleic acid delivery.

An example of the lipid-based transfection agent includes a cationic lipid. With a cationic lipid, nucleic acid-cationic lipid complexes are incorporated into cells via endocytosis and released into the cytoplasm, thus introducing the nucleic acid into cells (lipofection). Specifically, e.g. various commercially available reagents for lipofection may be employed.

An example of the polymer-based transfection agent includes e.g. a cationic polymer. When a cationic polymer comes in contact with a nucleic acid, a nucleic acid-polymer complex is formed, and the complex attaches to the cell membrane via electrostatic interaction and is incorporated into the cell via endocytosis. Specifically, a cationic peptide and a derivative thereof (such as polylysine and polyornithine) , a linear or branched-chain synthetic polymer (such as polybrene and polyethyleneimine), a polysaccharide-based introduction molecule (such as cyclodextrin and chitosan), a natural polymer (such as histone and collagen), as well as active and inactive dendrimers, and the like may be employed. Moreover, transfection agents employed in so-called nanoDDS, such as a transfection agent that employs a block copolymer that forms micellar nanoparticles and a transfection agent that employs carbon nanohorns can also be used for the present invention.

An example of the magnetic particle-based transfection agent includes a transfection agent that employs magnetic particles coated with cation molecules. A magnetic particle-based transfection agent carries out transfection by adhering the nucleic acid on the surface of magnetic particles, and then magnetically introducing the aforementioned magnetic particles into cells. Specifically, for example various commercially available magnetic particles for transfection may be employed.

Moreover, nucleic acid transfection agent that employs a generally available exosome or a transfection agent that utilizes viral proteins such as adenovirus can also be used for the present invention.

Further, in the present invention, a transfection agent comprising a peptide represented by amino acid sequences GGGGDD (G4D2) (SEQ ID NO. 14), GGGGGGDD (G6D2) (SEQ ID NO. 15), GGGGGGGGDD (G8D2) (SEQ ID NO. 16), GGGGGGGGGGDD (G10D2) (SEQ ID NO. 17), AAAAAAD (A6D) (SEQ ID NO. 18), AAAAAADD (A6D2) (SEQ ID NO. 19), AAAAAAK (A6K) (SEQ ID NO. 20), AAAAAAKK (A6K2) (SEQ ID NO. 21), VVVVVVD (V6D) (SEQ ID NO. 22), VVVVVVDD (V6D2) (SEQ ID NO. 23), VVVVVVK (V6K) (SEQ ID NO. 24), VVVVVVKK (V6K2) (SEQ ID NO. 25), LLLLLLD (L6D) (SEQ ID NO. 26), LLLLLLDD (L6D2) (SEQ ID NO. 27), LLLLLLK (L6K) (SEQ ID NO. 28), or LLLLLLKK (L6K2) (SEQ ID NO. 29) may be employed, and in particular AAAAAAD (A6D) or AAAAAAK (A6K) can be favorably employed. The effects of these peptides as transfection agents are disclosed in e.g. WO2010/024262.

Where administration steps or medical treatment methods are described herein, this description is to be understood as meaning that the invention relates to the provision of the pharmaceutical composition for use in such methods. The step of administration and the treatment method itself are not part of the invention.

The pharmaceutical composition for use in cancer therapy of the present invention can be used in combination with other anticancer agents well-known in the art. Other anticancer agents used in combination are not limited, and e.g. one or more anticancer agents selected from the group consisting of an alkylating agent, a platinum preparation, a metabolism antagonist, a topoisomerase inhibitor, a microtubular inhibitor, an anti-cancerous antibiotic, a molecular target drug, a hormone preparation, an immunomodulation drug, an interferon, an interleukin, a plant-derived anticancer agent, and a BRM preparation can be employed.

The aspects of combination use of the pharmaceutical composition for use in cancer therapy of the present invention and the other anticancer agents well-known in the art are not limited, and can be carried out by those skilled in the art (such as a physician) in various aspects according to the type of cancer to be the subject or therapeutic stage and the like. The pharmaceutical composition for use in cancer therapy of the present invention and the other anticancer agents may be administered to the subject at the same or different times. The pharmaceutical composition for use in cancer therapy of the present invention and the other anticancer agents may also be prepared as formulations comprising each and administered to a subject. The pharmaceutical composition for use in cancer therapy of the present invention and the other anticancer agents may also be prepared as a kit that separately comprises each.

An aspect of administering the pharmaceutical composition for use in cancer therapy of the present invention and the other anticancer agents at the same time may be e.g. an aspect of administering to a subject a formulation comprising the pharmaceutical composition for use in cancer therapy of the present invention and the other anticancer agents.

In the present invention, an aspect of administering the pharmaceutical composition for use in cancer therapy of the present invention and the other anticancer agents at different times may be e.g. an aspect of administering each of the pharmaceutical composition for cancer therapy of the present invention and the other anticancer agents with staggered time, and e.g. an aspect of administering the pharmaceutical composition for use in cancer therapy of the present invention and the other anticancer agents from different administration routes.

The administration route of the pharmaceutical composition for use in cancer therapy of the present invention is not limited, and may be systemic administration or topical administration. Administration routes can include e.g. oral administration including sublingual administration, parenteral administration such as inhalation administration, direct administration to target tissue by catheter or injection, intravenous administration including infusion, transdermal administration by patches and the like, suppositories, or administration by forced enteral nutrition employing a nasogastric tube, a nasointestinal tube, a gastrostomy tube, or enterostomy tube, and the like.

The dosage form of the pharmaceutical composition for use in cancer therapy of the present invention may be appropriately determined according to said administration route, and can include, but is not limited to, injections, infusions, tablets, capsules, fine granules, powders, liquids, solutions dissolved in syrups etc., patches, suppositories, and the like.

The subject for administering the pharmaceutical composition of the present invention is not limited, and e.g. the present invention can be employed for mammals (humans, pigs, cows, monkeys, baboons, dogs, cats, rats, mice, and the like). However, when it is unfavorable, humans can be removed from subjects.

The administration method of the pharmaceutical composition of the present invention to a subject (administration route, dosage, administration frequency per day, administration timing, and the like) is not limited, and can be appropriately determined by those skilled in the art (such as a physician) according to the health state of the subject, the extent of disease, the type of agent used in combination, and the like.

The terms used herein, except for those that are particularly defined, are employed for describing particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

The present invention will now be described in further detail with reference to Examples and Comparative Examples. However, the present invention can be embodied by various aspects, shall not be construed as being limited to the Examples described herein.

The Examples relating to miR-657 are Examples of the invention. Examples relating to other miRNAs, in particular miR-3140, miR-137, and miR-631 are Comparative Examples.

### Examples

### Experiment 1: Screening of miRNA with high cell aging inducibility

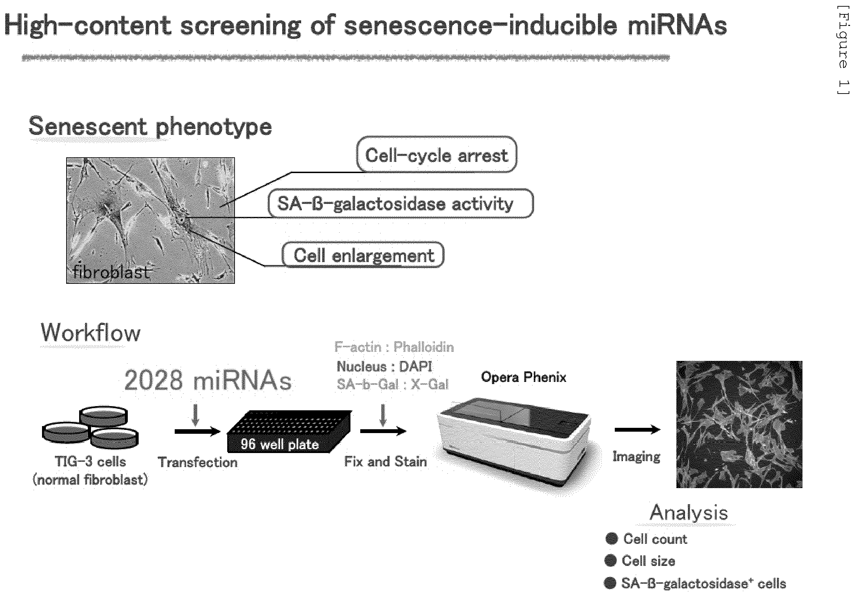

### (1) Transfection of all miRNAs into TIG-3 cells (Figure 1)

A total of 2028 types of miRNAs (mirVana; Ambion) were transfected into TIG-3 cells (human fibroblasts) with an auto dispenser Bravo (Agilent). Transfection was carried out by the following protocol.
1: To 70 µL of serum free medium (SFM) was added 0.35 µL of RNAiMAX (Invitrogen) .
2: The solution from 1 was added to a 96-well plate (µ-plate; ibidi) with Bravo.
3: To 2 was added 0.7 µL of miRNA (Stock Conc. 2 µM) with Bravo, and this was mixed by pipetting.
4: This was incubated at room temperature for 20 minutes.
5: A cell suspension diluted to 3.5 x 10⁴ cells/mL was dispensed at 70 µL each with Bravo.
6: This was incubated at 37°C under 5% CO₂ condition.

### (2-1) Staining of transfected cells

Setting the day of transfection as Day 0, staining was performed five days later in order to evaluate the number of cells and cell size. The protocol therefor is shown below.
1: Washing twice with PBS (-) was carried out.
2: 3.7% formalin solution was added, and this was incubated at room temperature for 10 minutes to fix the cells.
3: Washing twice with PBS (-) was carried out.
4: Staining solution was added, and this was incubated at room temperature for 30 minutes for staining.
5: Washing three times with PBS (-) was carried out.
6: The plate after completion of staining was subjected to full visual field photographing with an automatic photographing equipment Operetta (Perkin Elmer).
7: The photographs taken were subjected to quantitative analysis with an image analysis software Columbus (Perkin Elmer).

**[Table 5]**

| Table 1: Composition of the staining solution | | |
|---|---|---|
| Reagent name | 1mL | final Conc. |
| PBS(-) | 1mL | |
| Triton X-100 (NACALAI TESQUE) | 1µL | 0.1 % |
| BSA (Sigma-Aldrich) | 10 mg | 1 % |
| Alexa Fluor^{®} 488 Phalloidin (Thermo Fisher Scientific) | 4 µL | 0.8 Unit |
| DAPI (1mg/mL) (DOJINDO) | 0.1 µL | 0.1 µg/mL |

### (2-2) SA-β-galactosidase assay of transfected cells

Setting the day of transfection as Day 0, SA-β-galactosidase assay was performed on Day 7. The operating protocol is shown below.
1: Washing twice with PBS (-) was carried out.
2: 2% formalin solution was added, and this was incubated at room temperature for 5 minutes to fix the cells.
3: Washing twice with PBS (-) was carried out.
4: β-Gal staining solution was prepared at the time of use and added to the wells.
5: This was incubated at 37°C for 12 - 16 hours.
6: The plate after completion of staining was photographed with an automatic photographing equipment Opera (Perkin Elmer).

**[Table 6]**

| | | |
|---|---|---|
| Table 2: Composition of | β-Gal staining | solution |
| Reagent name | stock conc. | final conc. |
| citric acid / Na phosphate buffer *¹ | 0.2 M | 40mM |
| K₄{Fe(CN)₆}3H₂O | 100 mM | 5 mM |
| K₃{Fe(CN)₆} | 100 mM | 5 mM |
| NaCl | 5 M | 150 mM |
| MgCl₂·6H₂O H₂O | 1 M | 2 mM |
| X-gal *² | 20 mg/mL | 1 mg/mL |

| | | |
|---|---|---|
| *1: Prepared 50 that pH is 6.0. *2: X-gal (5-Bromo-4-chloro-3-indolyl-β-D-Galactopyranoside: Wako) was prepared at the time of use with N.N-dimethvl formamide. | | |

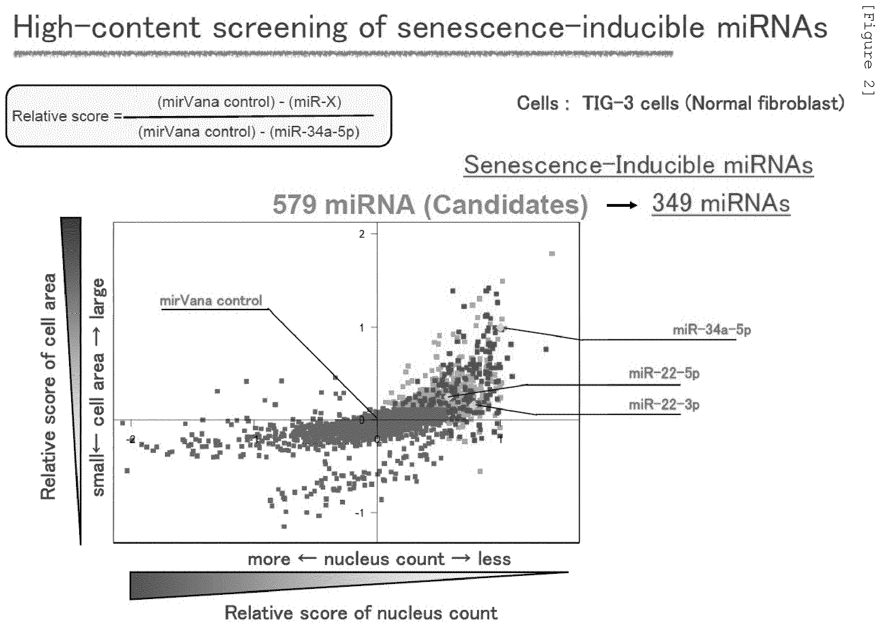

### (3) Analysis of the screening result (Figure 2)

Each of the values of the number of cells and cell size obtained were scored with the numeric values of random sequence miRNA which is the negative control and the numeric values of miR-34a-5p which is the positive control. The scoring method is shown below.

### Within each plate

1: The value of the positive control was subtracted from the value of the negative control.
2: The value of each miRNA was subtracted from the value of the negative control.
3: The value from 2 was divided by the value from 1 to obtain a score value.
4: A scatter diagram was drawn by taking the score value of the number of cells on the horizontal axis and the score value of the cell size on the vertical axis.
5: 579 types of miRNAs where at least one of the two showed a score value higher than the score values of miR-22-3p and miR-22-5p were identified as aging induction miRNA candidates.
6: Out of the 579 types of candidates, 349 types of miRNAs that induced activation of β-galactosidase which is a cell aging marker were identified as aging induction miRNAs.

### Experiment 2: Further screening employing cancer cell strains

The 349 types of aging induction miRNA (mirVana; Ambion) obtained from screening were transfected into various cancer cell strains (large intestine cancer cell strain HCT116 (p53 wildtype and p53 deletion), pancreatic cancer cell strains BxPC-3 and CFPAC-1, and tongue cancer cell strain HSC-4) with an auto dispenser Bravo (Agilent).

Transfection was carried out with the following protocol.
1: To 70 µL of serum free medium (SFM) was added 0.35 µL of RNAiMAX (Invitrogen) .
2: The solution from 1 was added to a 96-well plate (µ-plate; ibidi) with Bravo.
3: To 2 was added 0.7 µL of miRNA (Stock Conc. 200 nM) with Bravo, and this was mixed by pipetting.
4: This was incubated at room temperature for 20 minutes.
5: A cell suspension diluted to 3.5 x 10⁴ cells/mL was dispensed at 70 µL each with Bravo.
6: This was incubated at 37°C under 5% CO₂ condition.

Setting the day of transfection as Day 0, on Day 5 the cell survival rate was evaluated with PrestoBlue (Invitrogen). The operating protocol is shown below.
1. The medium was exchanged to a medium comprising PrestoBlue diluted 20-folds, and this was incubated at 37°C for 1 hour.
2. The fluorescence value (Ex/Em = 560 nm/590 nm) was measured with Enspire (Perkin Elmer).
3. The cell survival rate was determined by setting the fluorescence value obtained from the well with only the reagent as the background.

The above protocol was carried out for each cancer cell strain (large intestine cancer cell strain HCT116 (p53 wildtype and p53 deletion), pancreatic cancer cell strains BxPC-3 and CFPAC-1, and tongue cancer cell strain HSC-4), and the miRNAs that suppress cell proliferation more significantly than miR-34a-5p were sorted.

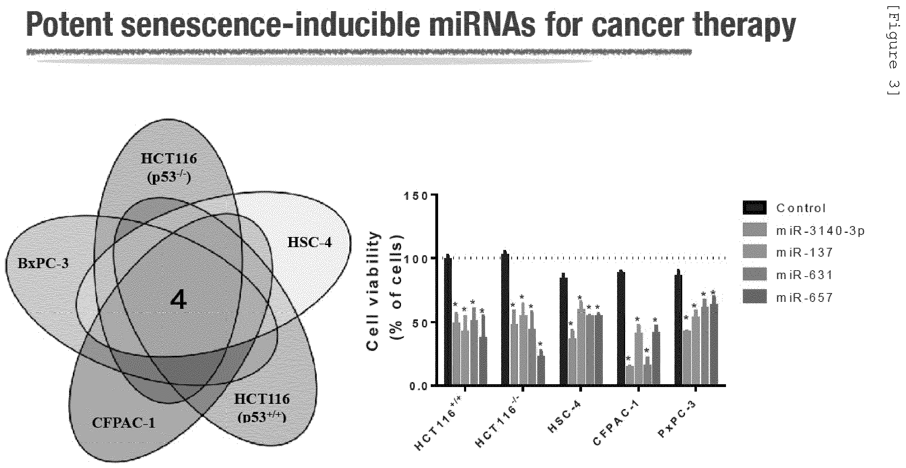

As a result of sorting the miRNAs that suppress cell proliferation more significantly than miR-34-5p commonly in all cell strains, four miRNAs (miR-137-3p, miR-631-5p, miR-657-3p, and miR-3140-3p) were found (Figure 3).

### Experiment 3. Confirmation of cell growth inhibition effect employing various cancer cell strains

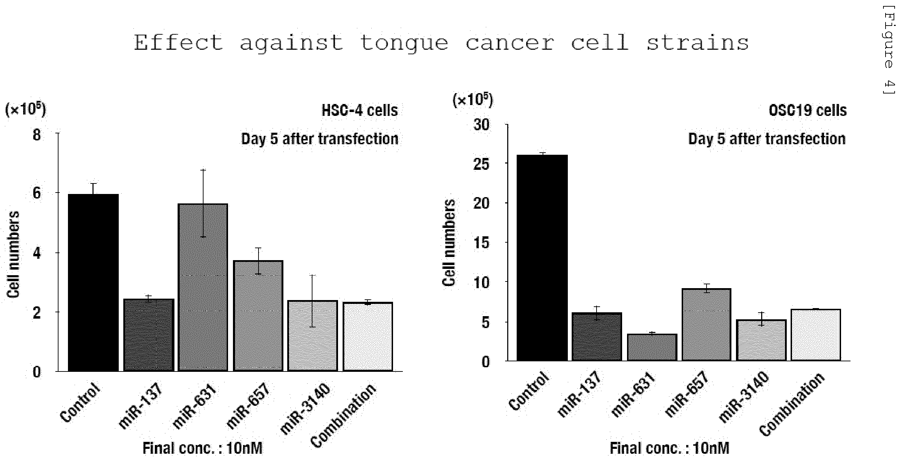

### (1) Tongue cancer cell strain (HSC-4 and OSC-19) (Figure 4)

Four miRNAs (miR-137-3p, miR-631-5p, miR-657-3p, and miR-3140-3p) were transfected into tongue cancer cell strains (HSC-4 and OSC-19), and cell proliferation was observed. Transfection was carried out with the following protocol.
1: To a 35 mm dish were added 500 µL of serum free medium (SFM) and 5 µL of RNAiMAX (Invitrogen).
2: To the solution from 1 was added 1 µL each of nucleic acids (Control, miR-137-3p, miR-631-5p, miR-657-3p, miR-3140-3p, and a mixture of equal amounts of the four miRNAs (final concentration 10 nM).
3: This was incubated at room temperature for 20 minutes.
4: A cell suspension diluted to 6.7 x 10⁴ cells/mL was added to the 35 mm dish at 1.5 mL each.
5: This was incubated at 37°C under 5% CO₂ condition.
6: The cells were counted five days after transfection.

As shown in Figure 4, the four miRNAs showed cell growth inhibition effect in both tongue cancer cell strains.

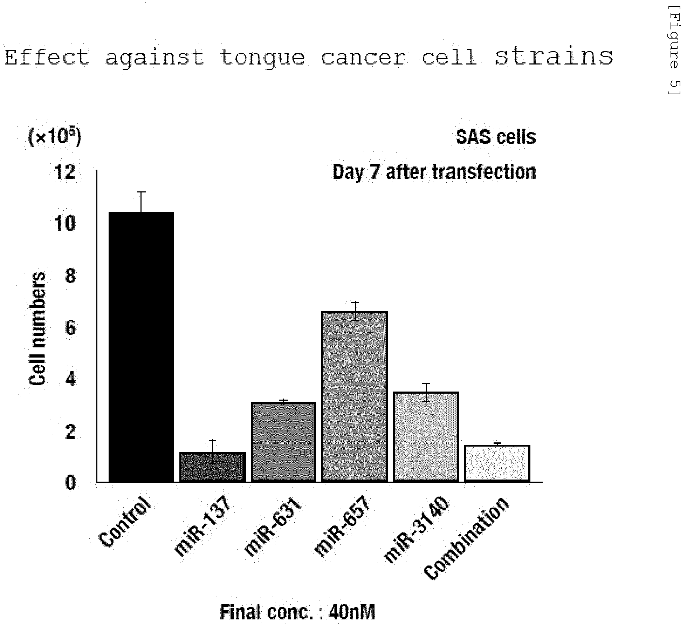

### (2) Tongue cancer cell strain (SAS) (Figure 5)

Four miRNAs (miR-137-3p, miR-631-5p, miR-657-3p, and miR-3140-3p) were transfected into a tongue cancer cell strain (SAS), and cell proliferation was observed. Transfection was carried out with the following protocol.
1: To a 35 mm dish were added 500 µL of serum free medium (SFM) and 5 µL of RNAiMAX (Invitrogen).
2: To the solution from 1 was added 4 µL each of the miRNA solutions (see Table 3) (final concentration 10 nM).
3: This was incubated at room temperature for 20 minutes.
4: 1.5 mL of a cell suspension diluted to 6.7 x 10⁴ cells/mL was added to the 35 mm dish.
5: This was incubated at 37°C under 5% CO₂ condition.
6: Operations from 1-3 were repeated two days after transfection.
7: The solutions prepared in 6 were each added to the dish cultured in 5.
8: The cells were counted seven days after the first transfection.

As shown in Figure 5, any of the four miRNAs showed cell growth inhibition effect against tongue cancer cell strain SAS.

**[Table 7]**

| Table 3: miRNA dosage | | | | | | |
|---|---|---|---|---|---|---|
| Stock(20µM) | Control | miR 137 3p | miR 631 5p | miR 657 3p | miR 3140 3p | Combination |
| miR Control | 4µL | 3µL | 3µL | 3µL | 3µL | |
| miR 137 3p | | 1µL | | | | 1µL |
| miR 631 5p | | | 1µL | | | 1µL |
| miR 657 3p | | | | 1µL | | 1µL |
| miR 3140 3p | | | | | 1µL | 1µL |
| Total | 4µL | 4µL | 4µL | 4µL | 4µL | 4µL |

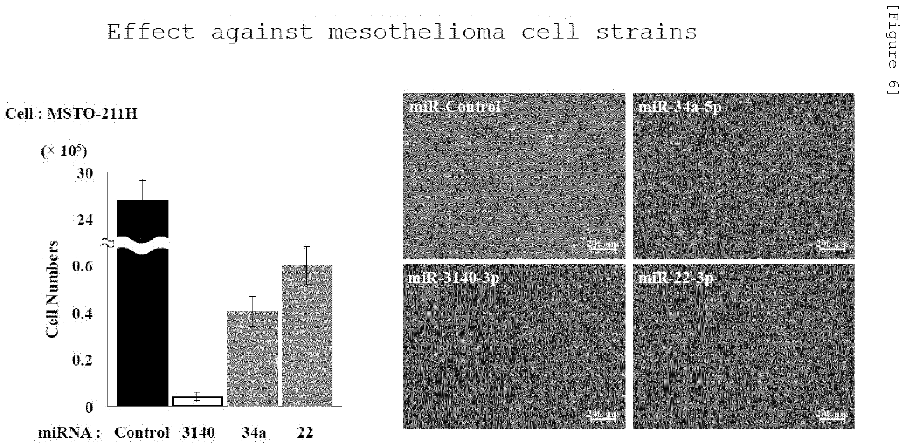

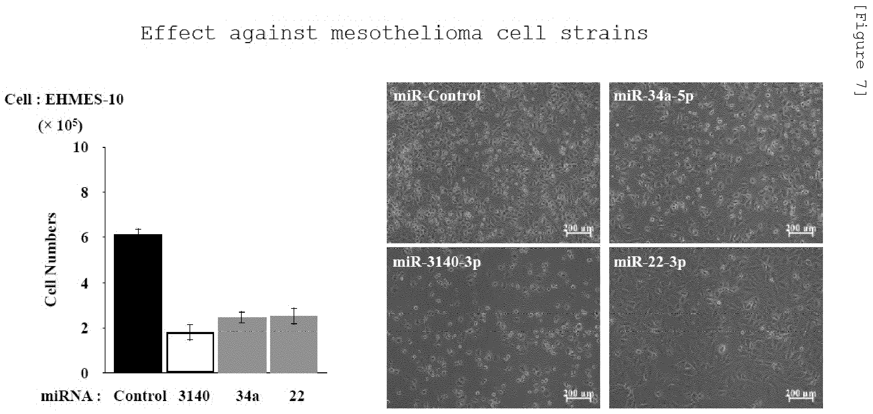

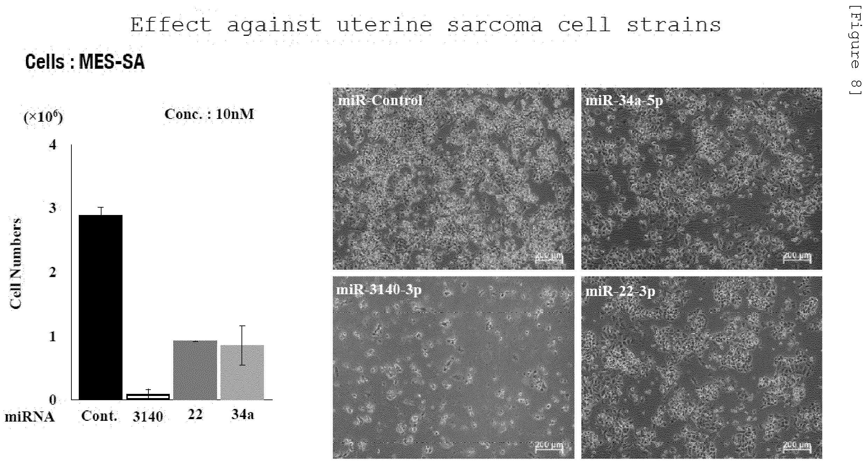

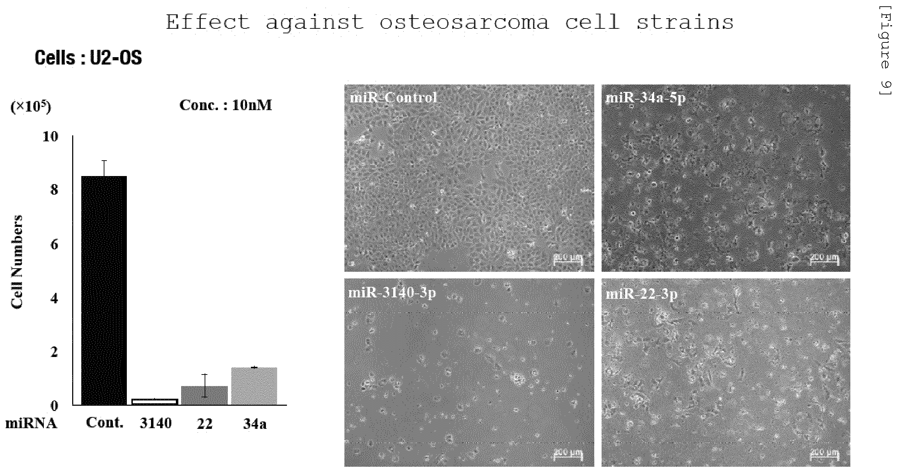

### (3) Malignant pleural mesothelioma cell strains, uterine sarcoma cell strain, and osteosarcoma cell strain (Figures 6-9)

Among the above four miRNAs, miR-3140-3p which had particularly high cell growth inhibition effect was transfected in each of malignant pleural mesothelioma cell strains (MSTO-211H and EHMES-10), uterine sarcoma cell strain (MES-SA), and osteosarcoma cell strain (U2-OS), and cell proliferation was observed. As comparison subjects, miR-22-3p and miR-34a-5p which showed cell growth inhibition effect in prior research were employed. Transfection was carried out with the following protocol.
1: To a 35 mm dish were added 500 µL of serum free medium (SFM) and 5 µL of RNAiMAX (Invitrogen).
2: To the solution from 1 was added 1 µL each of miR-Control or miR-3140-3p, miR-22-3p, and miR-34a-5p (stock conc. 20 µM) (final concentration 10 nM).
3: This was incubated at room temperature for 20 minutes.
4: 1.5 mL of a cell suspension diluted to 6.7 x 10⁴ cells/mL was added to the 35 mm dish.
5: This was incubated at 37°C under 5% CO₂ condition.
6: The cells were counted four days after transfection.

As shown in Figures 6-9, in any of the cell strains, miR-3140-3p showed extremely high cell growth inhibition effect compared to prior art miR-22-3p and miR-34a-5p.

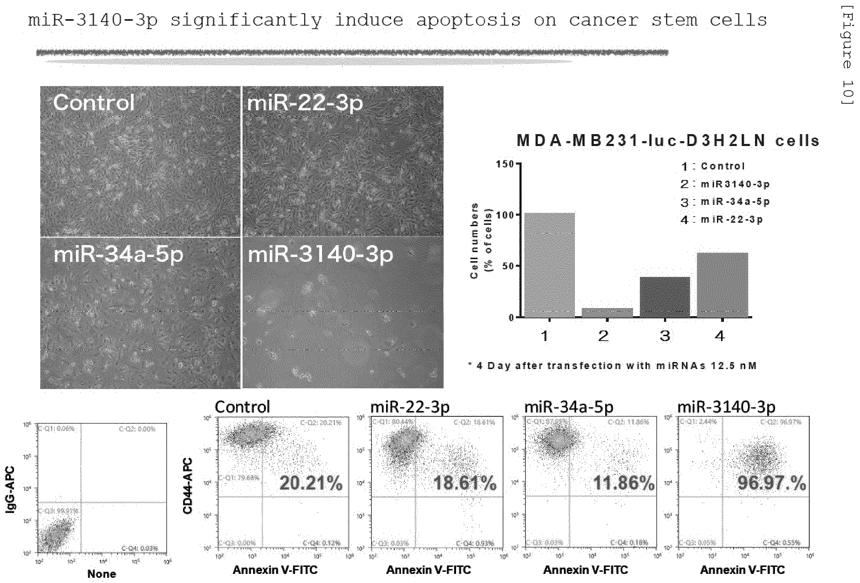

### (4) Breast cancer stem cell strain (MDA-MB231-luc-D3H2LN cells) (Figure 10)

miR-3140-3p was transfected in a highly metastatic cancer cell strain of breast cancer (MDA-MB231-luc-D3H2LN cells), and cell proliferation was observed. As comparison subjects, miR-22-3p and miR-34a-5p which showed cell growth inhibition effect in prior research were employed. Transfection was carried out with the following protocol.
1: To a 35 mm dish were added 500 µL of serum free medium (SFM) and 5 µL of RNAiMAX (Invitrogen).
2: To the solution from 1 was added 12.5 µL each of miR-Control or miR-3140-3p, miR-22-3p, and miR-34a-5p (stock conc. 2 µM) (final concentration 12.5 nM).
3: This was incubated at room temperature for 20 minutes.
4: 1.5 mL of a cell suspension diluted to 6.7 x 104 cells/mL was added to the 35 mm dish.
5: This was incubated at 37°C under 5% CO2 condition.
6: The cells were counted four days after transfection.

Moreover, by the protocol below, the expression of apoptosis marker Annexin V in breast cancer cells transfected with miR-3140-3p was analyzed.
1: Transfection of miR-3140-3p was performed with a protocol similar to that described above.
2: Six days after transfection, cells were recovered together with the supernatant.
3: Samples for FACS were prepared according to the protocol of the Annexin V assay kit.
4: Cells stained by Annexin V-FITC were stained with an antibody against cancer stem cell marker CD44 (eBioScience).
5: The prepared samples were analyzed with Cell Sorter (SONY).

As shown in Figure 10 top, miR-3140-3p showed extremely high cell growth inhibition effect against breast cancer stem cell strain (MDA-MB231-luc-D3H2LN cells).

Moreover, as shown in Figure 10 bottom, in breast cancer stem cells at six days after transfection of miR-3140-3p, apoptosis marker Annexin V was highly positive (96.97%). In other words, it was shown that miR-3140-3p significantly induces apoptosis against cancer stem cells.

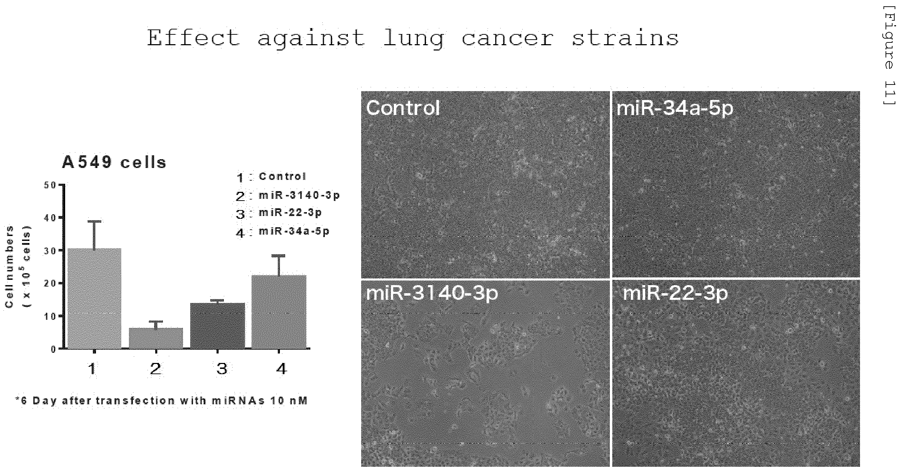

### (5) Lung cancer cell strain (A549) (Figure 11)

miR-3140-3p was transfected into lung cancer cell strain (A549), and cell proliferation was observed. As comparison subjects, miR-22-3p and miR-34a-5p which showed cell growth inhibition effect in prior research were employed. Transfection was carried out with the following protocol.
1: To a 35 mm dish were added 500 µL of serum free medium (SFM) and 5 µL of RNAiMAX (Invitrogen).
2: To the solution from 1 was added 1 µL each of miR-Control or miR-3140-3p, miR-22-3p, and miR-34a-5p (stock conc. 20 µM) (final concentration 10.0 nM).
3: This was incubated at room temperature for 20 minutes.
4: 1.5 mL of a cell suspension diluted to 6.7 x 10⁴ cells/mL was added to the 35 mm dish.
5: This was incubated at 37°C under 5% CO2 condition.
6: The cells were counted six days after transfection.

As shown in Figure 11, miR-3140-3p showed extremely high cell growth inhibition effect against lung cancer cell strain (A549).

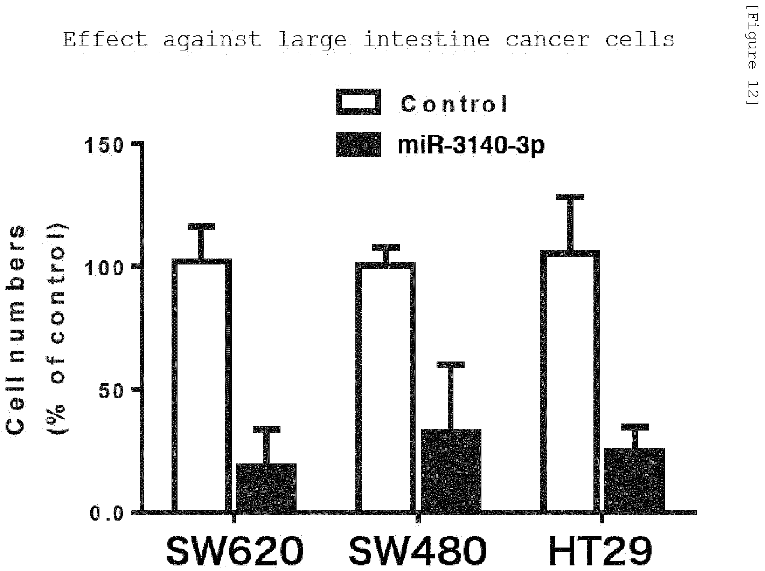

### (6) Large intestine cancer cell strains (SW620, SW480, and HT29) (Figure 12)

miR-3140-3p was transfected into large intestine cancer cell strains (SW620, SW480, and HT29), and cell proliferation was observed. Transfection was carried out with the following protocol.
1: To a 35 mm dish were added 500 µL of serum free medium (SFM) and 5 µL of RNAiMAX (Invitrogen).
2: To the solution from 1 was added 12.5 µL each of miR-Control or miR-3140-3p, miR-22-3p, and miR-34a-5p (stock conc. 2 µM) (final concentration 10.0 nM).
3: This was incubated at room temperature for 20 minutes.
4: 1.5 mL of a cell suspension diluted to 6.7 x 104 cells/mL was added to the 35 mm dish.
5: This was incubated at 37°C under 5% CO2 condition.
6: The cells were counted seven days after transfection.

Results are shown in Figure 12. Setting the number of control introduction cells as 100%, the survival rate of cells in which miR-3140-3p was introduced was shown in %. As shown in Figure 12, miR-3140-3p showed extremely high cell growth inhibition effect against any of the large intestine cancer cell strains.

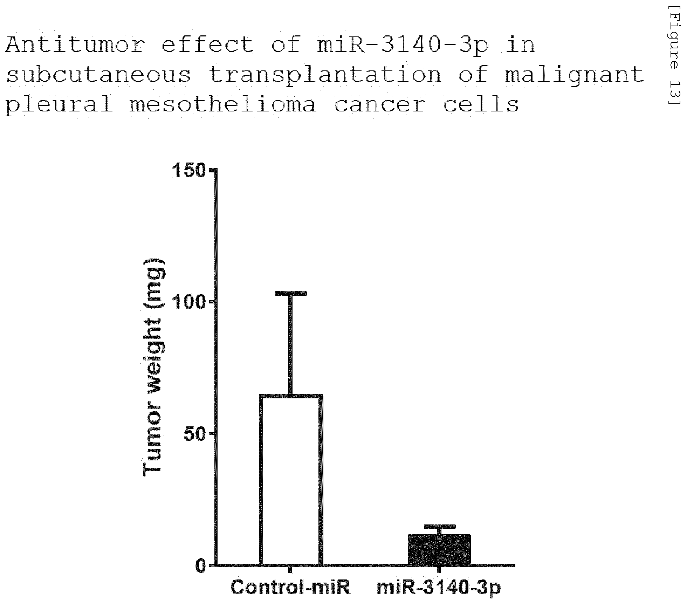

### Experiment 4. Antitumor effect of a comparative miRNA in vivo (malignant pleural mesothelioma cells) (Figure 13)

In order to verify the antitumor effect of a comparative miRNA *in vivo,* experiments was performed with laboratory animals transplanted with malignant pleural mesothelioma cells.

### (1) Preparation of cells

Malignant pleural mesothelioma cells MSTO-211H cells were used.
1: The cells on the dish were washed twice with PBS (-).
2: The cells were detached with trypsin.
3: This was suspended in a medium, and cells were counted.
4: Centrifugation at a condition of 1000 rpm for 3 min was performed to pellet down.
5: The cells were resuspended in PBS (-) in order to obtain 2.0 x 10⁷ cells/mL.

### (2) Cell transplantation to mice

Six weeks-old C-B-17/Icr-scid/scid Jcl (SCID mouse) were used as mice. SCID mice were subcutaneously administered 100 µL of the prepared cell suspension, and the cells were allowed to settle.

### (3) miRNA administration

Negative control which is the control sequence and miR-3140-3p which is the comparative miRNA were employed. A6K (from 3D Matrix) was employed as the nucleic acid delivery reagent. The administration of miRNA to mice was carried out with the following protocol.
1: 100 µM of the nucleic acid (miRNA) was diluted with 10% saline and sterilized water in order to obtain 71.4 µM.
2: 1% A6K solution was sonicated for 5 minutes before use.
3: The diluted nucleic acid and the 1% A6K solution were mixed at a proportion of 1:1 to give the administration nucleic acid.
4: SCID mice were subcutaneously administered (tumor site) 50 µL each of the administration nucleic acid.

### (5) Evaluation

The experimental results were evaluated with the following protocol.
1: From four days after transplantation, the nucleic acid was administered every one or two days.
2: A total of 13 administrations were performed, and 34 days after transplantation was set as the endpoint.
3: Mice were dissected, and the subcutaneous tumor was resected and weighed.

Experimental results are shown in Figure 13 (n=3). As shown in Figure 13, compared to the control group, the tumor weight was significantly lower in mice in which miR-3140-3p was introduced at the tumor site.

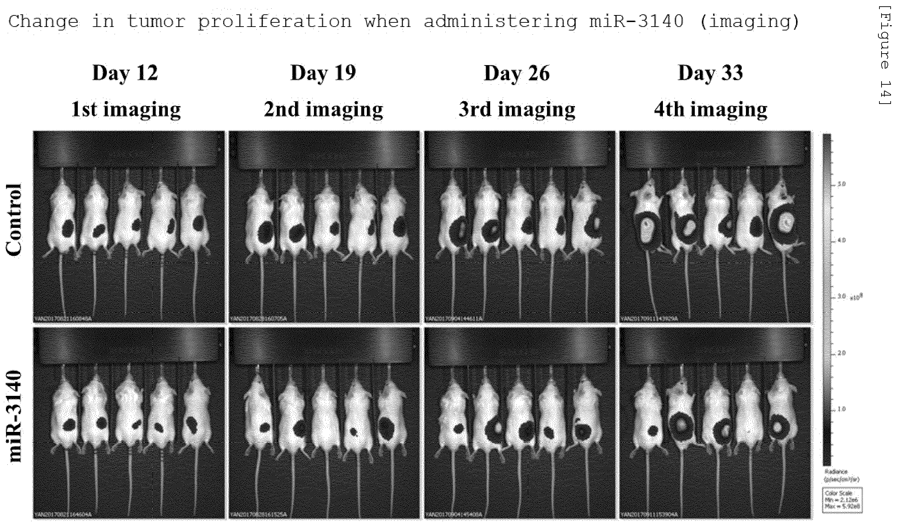

### Experiment 5. Antitumor effect of a comparative miRNA in vivo (malignant pleural mesothelioma cells) (Figure 14-16)

### (1) Preparation of cells

Malignant pleural mesothelioma cells EHMES-10 cells were used.
1: The cells on the dish were washed twice with PBS (-).
2: The cells were detached with trypsin.
3: This was suspended in a medium, and cells were counted.
4: Centrifugation at a condition of 1000 rpm for 3 min was performed to pellet down.
5: The cells were resuspended in PBS (-) in order to obtain 2.0 x 10⁷ cells/mL.

### (2) Cell transplantation to mice

Six weeks-old C-B-17/Icr-scid/scid Jcl (SCID mouse) were used as mice. SCID mice were subcutaneously administered 100 µL of the prepared cell suspension, and the cells were allowed to settle.

### (3) miRNA administration

Negative control which is the control sequence and miR-3140-3p which is the comparative miRNA were employed. A6K (from 3D Matrix) was employed as the nucleic acid delivery reagent. The administration of miRNA to mice was carried out with the following protocol.
1: 100 µM of the nucleic acid (miRNA) was diluted with 10% saline and sterilized water in order to obtain 71.4 µM.
2: 1% A6K solution was sonicated for 5 minutes before use.
3: The diluted nucleic acid and the 1% A6K solution were mixed at a proportion of 1:1 to give the administration nucleic acid.
4: SCID mice were subcutaneously administered (tumor site) 50 µL each of the administration nucleic acid.

### (5) Evaluation

The experimental results were evaluated with the following protocol.
1: From two days after transplantation, the nucleic acid was administered every one or two days.
2: A total of 13 administrations were performed, and 33 days after transplantation was set as the endpoint.
3: At 12, 19, 26, and 33 days after transplantation, luciferin was intraperitoneally administered for imaging in order to trace the tumor size.
4: Mice were dissected at the endpoint, and the subcutaneous tumor was resected and weighed.

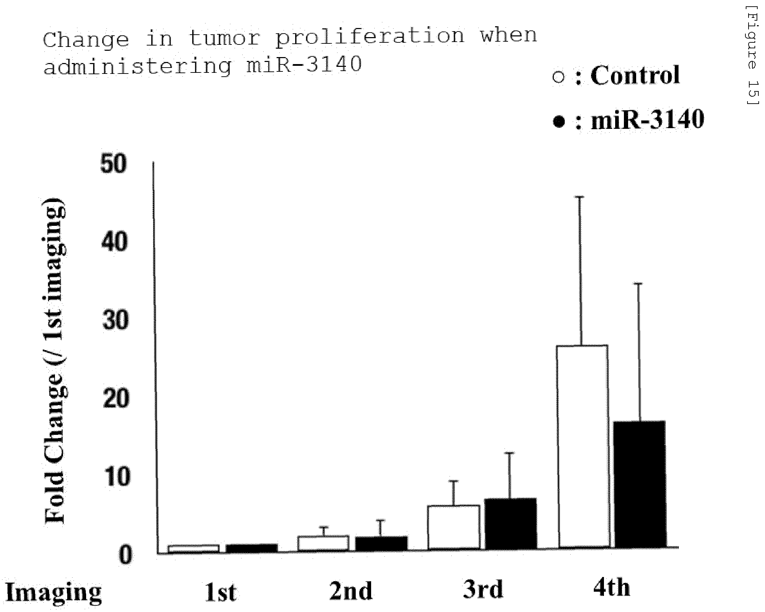

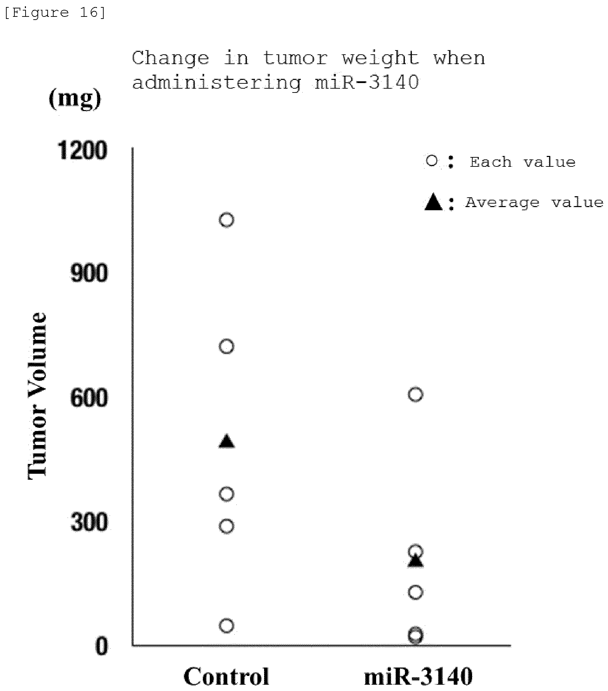

Imaging analysis results of tumor tissues at 12, 19, 26, and 33 days after tumor cell transplantation are shown in Figure 14 and Figure 15. As shown in Figures 14 and Figure 15, compared to the control group, expansion of the tumor was suppressed in mice in which miR-3140-3p was introduced at the tumor site. Moreover, a similar result was shown in Figure 16 which compares the tumor weight at endpoint.

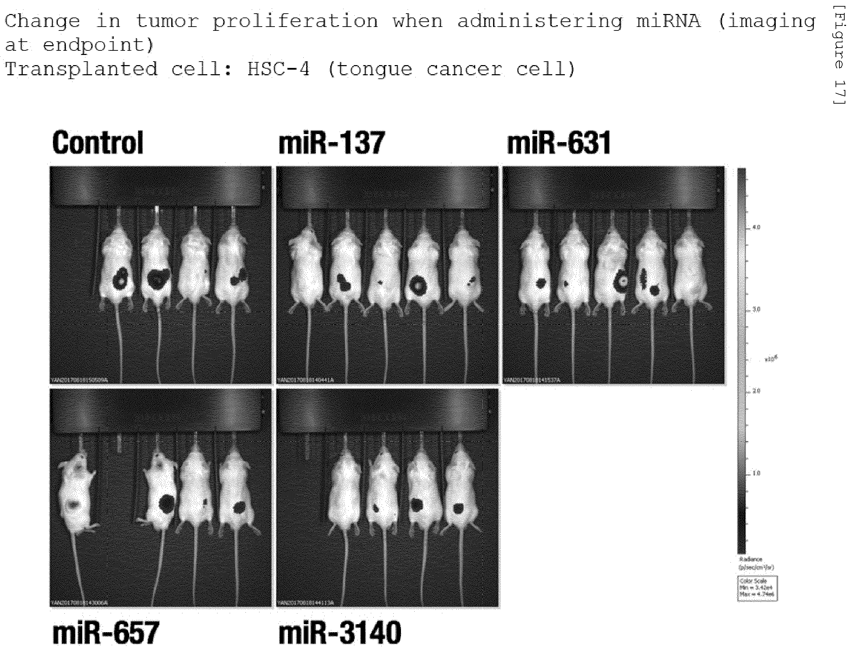

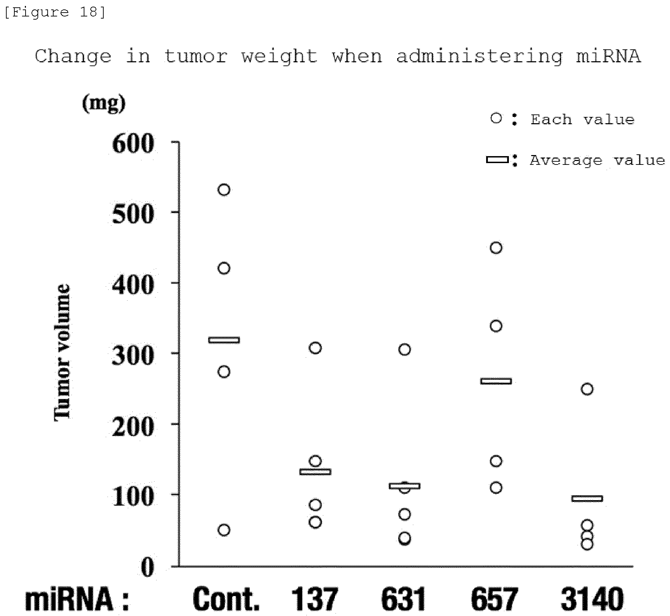

### Experiment 6. Antitumor effect of the miRNA of the present invention and comparative miRNAs in vivo (tongue cancer cell) (Figures 17 and 18)

### (1) Preparation of cells

Tongue cancer cell strain HSC-4 cells were used.
1: The cells on the dish were washed twice with PBS (-).
2: The cells were detached with trypsin.
3: This was suspended in a medium, and cells were counted.
4: Centrifugation at a condition of 1000 rpm for 3 min was performed to pellet down.
5: The cells were resuspended in PBS (-) in order to obtain 2.0 x 10⁷ cells/mL.

### (2) Cell transplantation to mice

Six weeks-old C-B-17/Icr-scid/scid Jcl (SCID mouse) were used as mice. SCID mice were subcutaneously administered 100 µL of the prepared cell suspension, and the cells were allowed to settle.

### (3) miRNA administration

Negative control which is the control sequence and miR-3140-3p, miR-137, and miR-631, which are comparative miRNAs, and miR-657 which is the miRNA of the present invention were employed. A6K (from 3D Matrix) was employed as the nucleic acid delivery reagent. The administration of miRNA to mice was carried out with the following protocol.
1: 100 µM of the nucleic acid (miRNA) was diluted with 10% saline and sterilized water in order to obtain 71.4 µM.
2: 1% A6K solution was sonicated for 5 minutes before use.
3: The diluted nucleic acid and the 1% A6K solution were mixed at a proportion of 1:1 to give the administration nucleic acid.
4: SCID mice were subcutaneously administered (tumor site) 50 µL each of the administration nucleic acid.

### (5) Evaluation

The experimental results were evaluated with the following protocol.
1: From three days after transplantation, the nucleic acid was administered every one or two days.
2: A total of 11 administrations were performed, and 28 days after transplantation was set as the endpoint.
3: At 7, 14, 21, and 28 days after transplantation, luciferin was intraperitoneally administered for imaging in order to trace the tumor size.
4: Mice were dissected at the endpoint, and the subcutaneous tumor was resected and weighed.

Imaging analysis results of tumor tissues after tumor cell transplantation are shown in Figure 17. As shown in Figure 17, compared to the control group, expansion of the tumor was suppressed in mice in which the miRNA of the present invention or comparative miRNAs was introduced at the tumor site. Moreover, a similar result was shown in Figure 18 which compares the tumor weight at endpoint.

From the above results, it was shown that the miRNA of the present invention also exerts extremely strong antitumor effect *in vivo*.

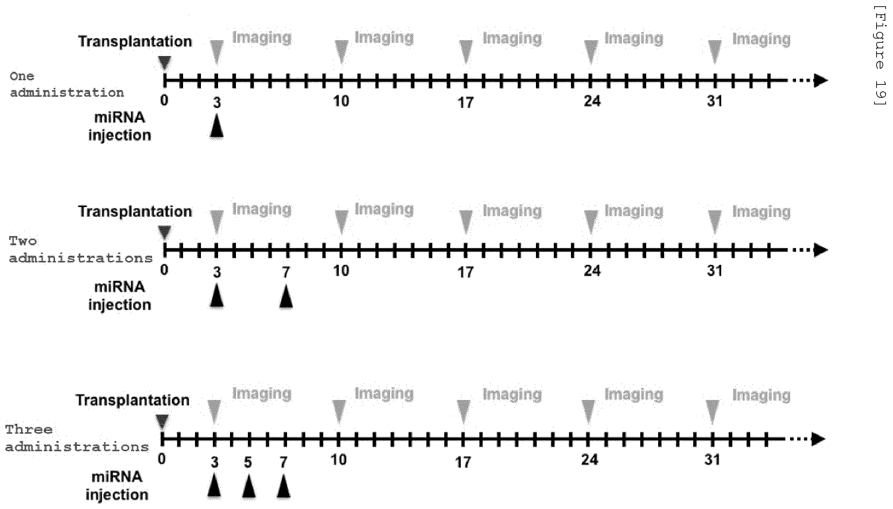

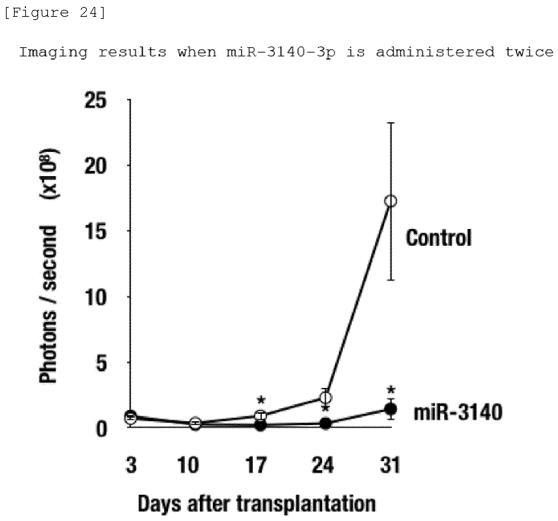

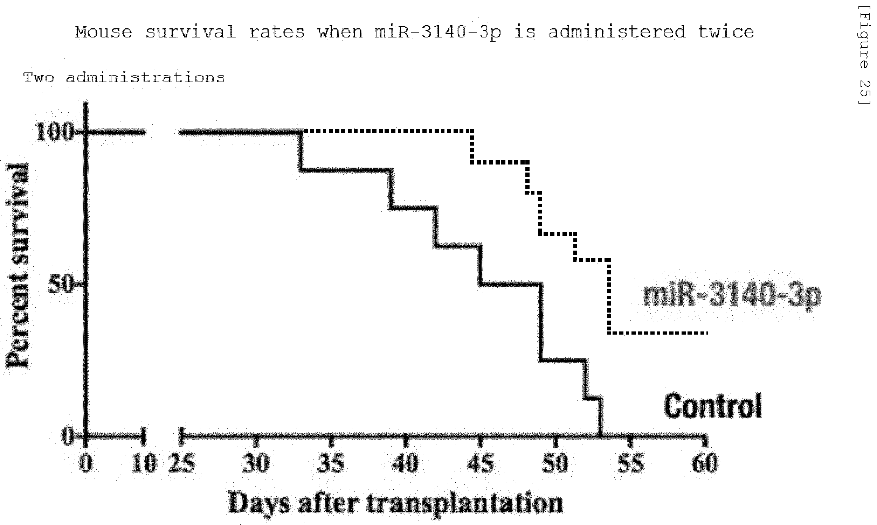

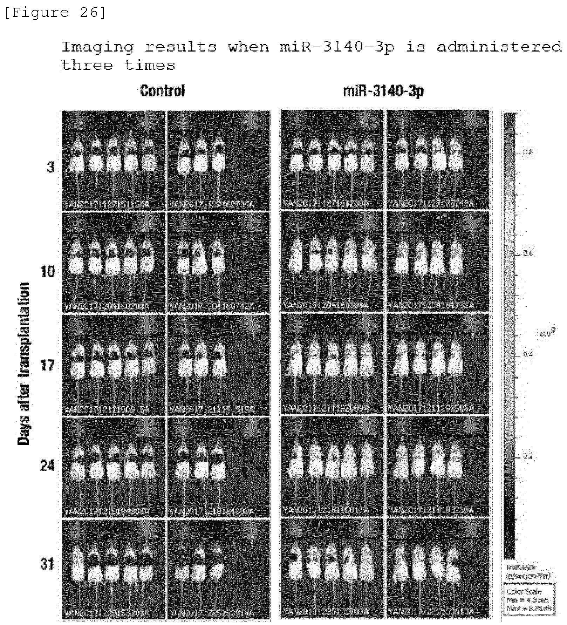

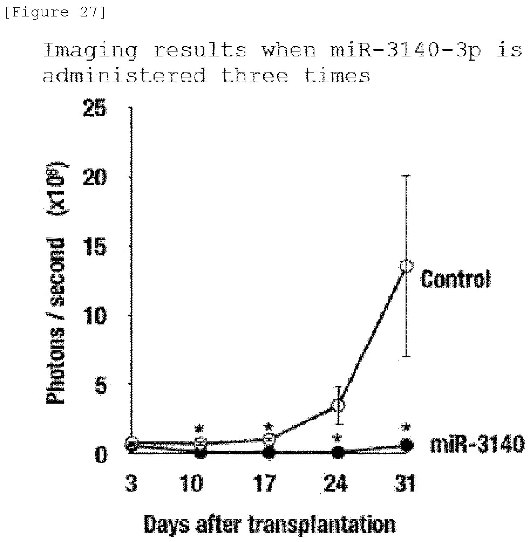

### Experiment 7. Antitumor effect of a comparative miRNA in vivo (malignant pleural mesothelioma cells) (Figures 19-28)

The tumor suppression effect of miR-3140-3p (a comparative miRNA) *in vivo* was investigated with intrathoracic orthotopic transplantation model mouse.

Six weeks-old male mice (C-B-17/Icr-scid/scid Jcl) were used as mice. Malignant pleural mesothelioma cell strain EHMES-10 which expresses the luciferase gene was used as the tumor cell.

The experiment protocol is shown below.
1: Mice were intraperitoneally administered 0.1 mL per 10 g of body weight of a mixed anesthetic drug of medetomidine hydrochloride, midazolam, and butorphanol tartrate.
2: After anesthesia, mouse chest hair was shaved, and an incision was made in the epidermis with scissors.
3: In the mouse pleural cavity 100 µL of tumor cells (3 x 10⁷ cells/mL) was transplanted with a 27 G syringe for insulin.
4: Three days after transplantation, imaging of tumor cells was performed with IVIS Spectrum CT *In vivo* Imaging System.
5: After imaging, grouping was performed with successfully transplanted mice.
6: Mice were intraperitoneally administered 0.1 mL per 10 g of body weight of a mixed anesthetic drug of medetomidine hydrochloride, midazolam, and butorphanol tartrate to anesthetize the mice.
7: After anesthesia, 100 µL of miRNA/A6K mixture was administered in the mouse pleural cavity.
8: Imaging was performed every week from the first imaging, and tumor expansion was observed.
9: The time and date of death of the mice was recorded, and mice survival rate was calculated.

Administration and imaging schedule is shown in Figure 19. The mixed anesthetic drug of medetomidine hydrochloride, midazolam, and butorphanol tartrate was prepared as in Table 4 below. The miRNA/A6K mixture was prepared as in Table 5. Moreover, the control group was administered the RNA shown in Table 6 instead of the comparative miRNA of the present document.

Note that in order to improve expression efficiency, miRNA was administered as a double-strand in combination with a complementary strand comprising partial mismatch.

### [Table 8]

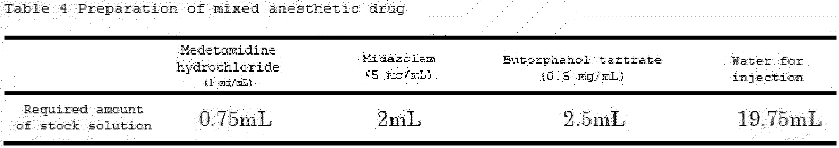

### [Table 9]

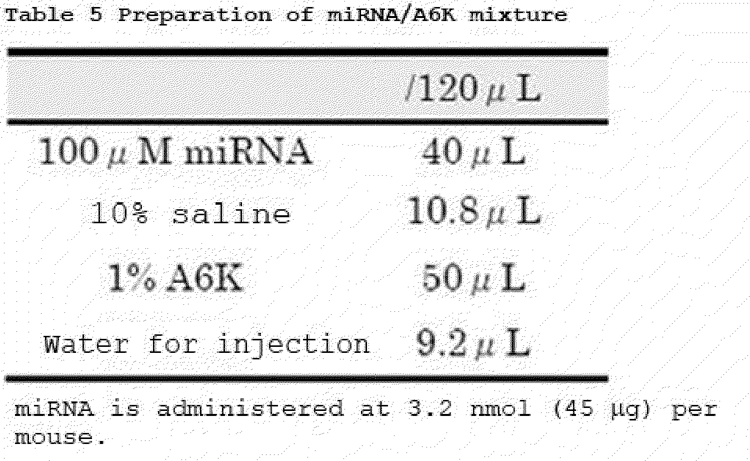

### [Table 10]

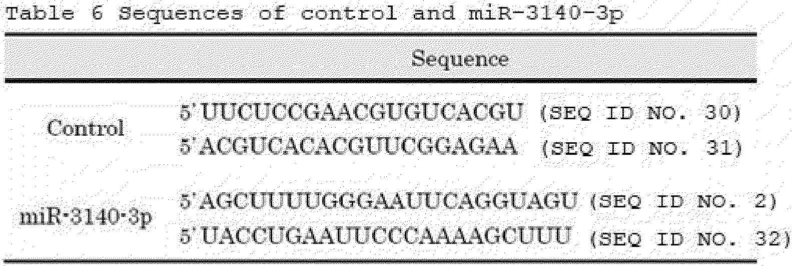

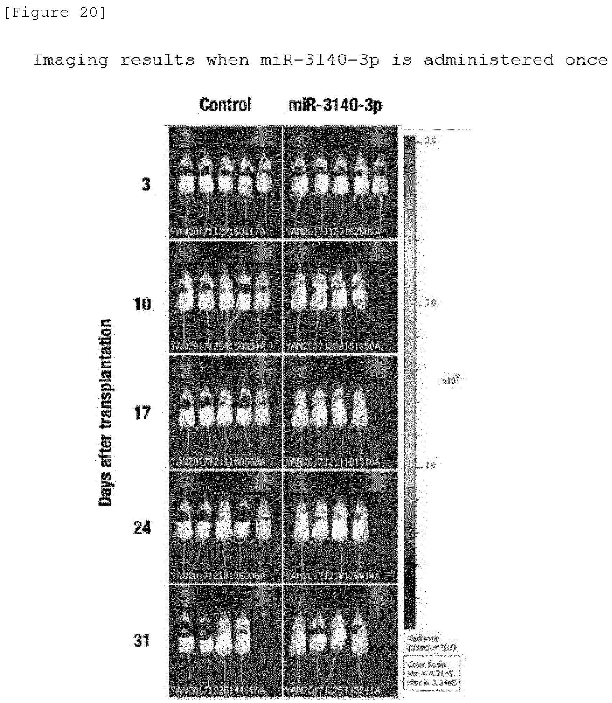

Experimental results are shown in Figure 20 to Figure 28.

In the group with one administration of miR-3140-3p, tumor reduction was seen by the second imaging (Day 10 after transplantation), and the effect had persisted until the fourth imaging (Day 24 after transplantation) (Figure 20).

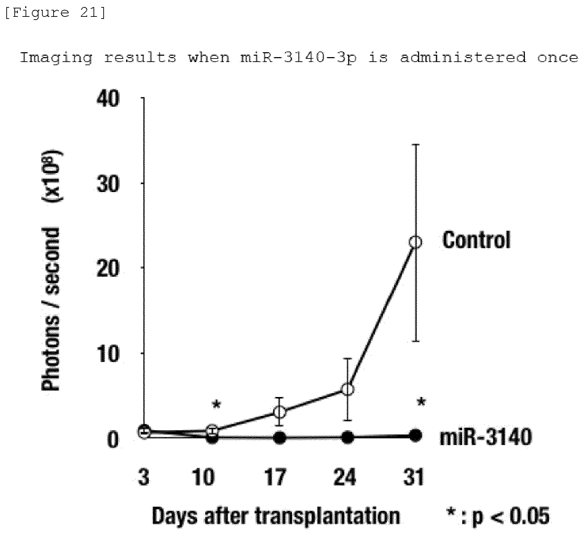

The imaging results were digitized and graphed, and it was shown that miR-3140-3p significantly suppressed tumor expansion (Figure 21).

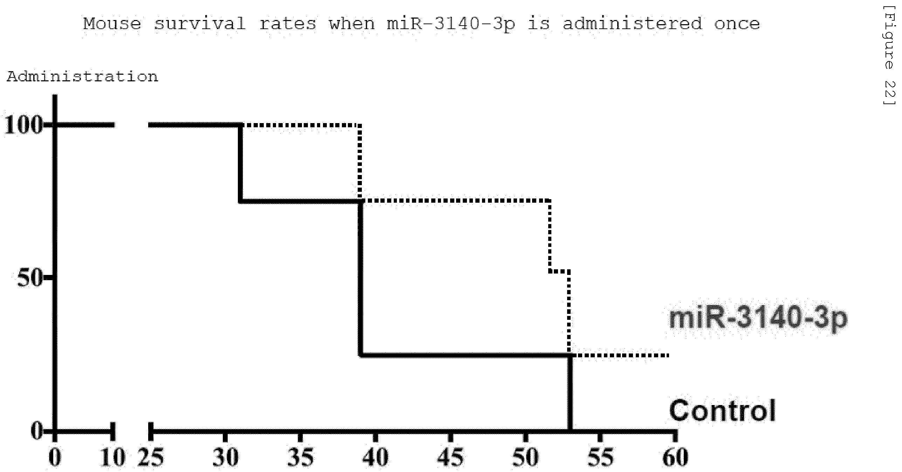

By comparing the survival rates of mice, it was observed that the survival rates of mice had improved in the miR-3140-3p administration group (Figure 22).

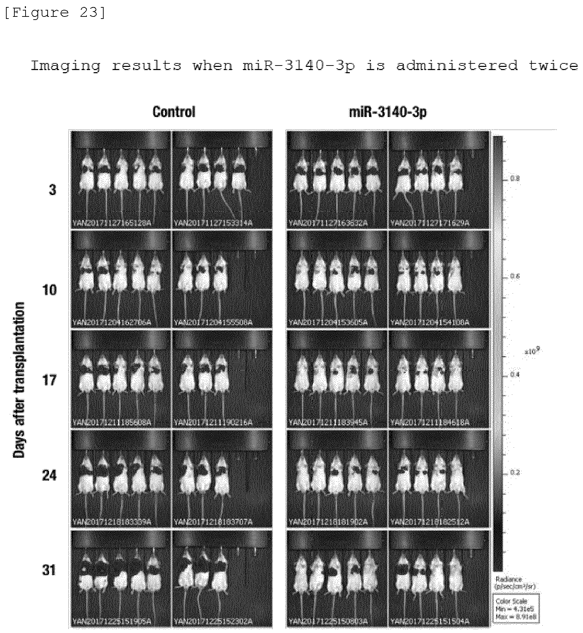

It was shown that similarly to the group with one administration, miR-3140-3p also significantly suppressed malignant pleural mesothelioma in the groups with two and three administrations of miR-3140-3p (Figures 23-28).

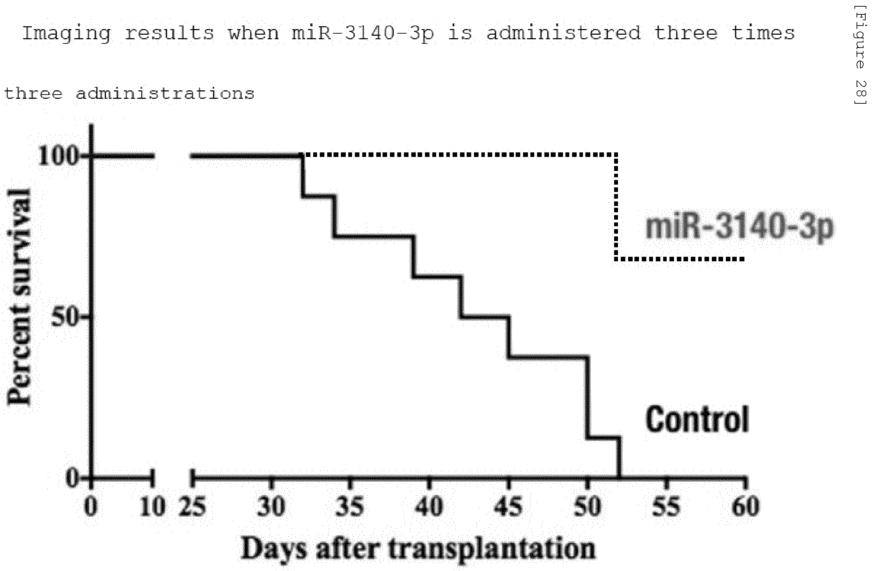

The survival rates of mice were more improved in the group with three administrations of miR-3140-3p (Figure 28).

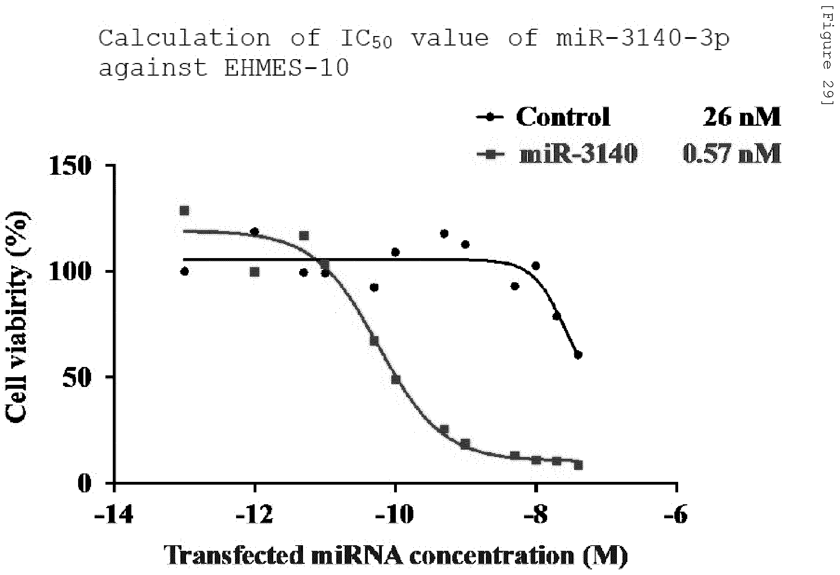

### Experiment 8. Calculation of IC₅₀ value (Figure 29)

The IC₅₀ value of miR-3140-3p was calculated with malignant pleural mesothelioma cell EHMES-10.

The protocol therefor is shown below.
1: To 25 µL of serum free medium (SFM) was added 0.25 µL of RNAiMAX (Invitrogen) .
2: miR-Control and miR-3140-3p were serially diluted so that the final concentrations will be 40 nM, 20 nM, 10 nM, 5 nM, 1 nM, 500 pM, 100 pM, 50 pM, 10 pM, 5 pM, and 1 pM, and mixed with SFM/RNAiMAX complex.
3: This was incubated at room temperature for 20 minutes.
4: 75 µL of each of cells at 6.7 x 10⁴ cells/mL were added to each well.
5: This was incubated at 37°C under 5% CO₂ condition.
6: Five days after transfection, the survival rates of cells were investigated with Cell Counting Kit 8 (DOJINDO). The protocol therefor is shown below.
   (i) Cell Counting Kit was diluted 10-folds with a medium.
   (ii) 200 µL of each of the diluted Cell Counting Kit was added to each well.
   (iii) This was incubated at 37°C under 5% CO₂ condition for 1 hour.
   (iv) The values at 450 nm/600 nm were measured with a plate reader.

Note that the same control RNA as that used in Experiment 7 was employed as the control.

Results are shown in Figure 29. As shown in Figure 29, it was revealed that miR-3140-3p shows growth inhibition effect against malignant pleural mesothelioma cell strain EHMES-10 even at a very low concentration. Calculation of the IC₅₀ value gave a final concentration of 0.57 nM.

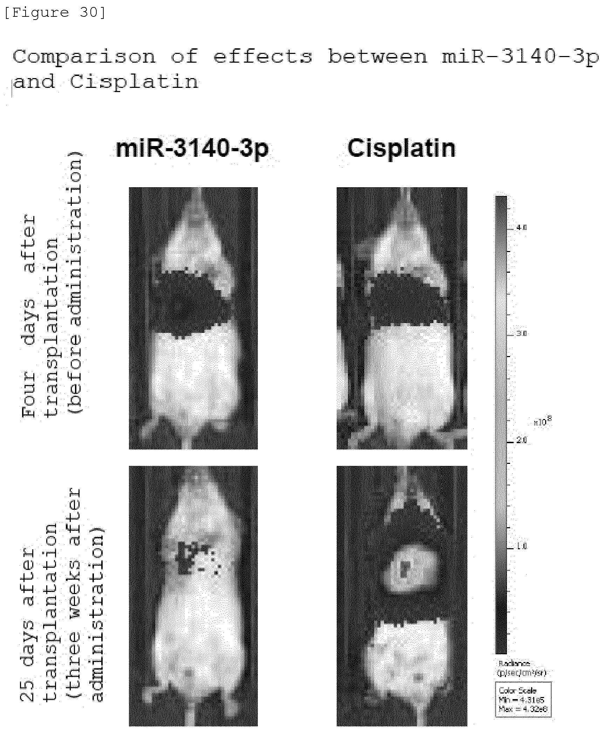

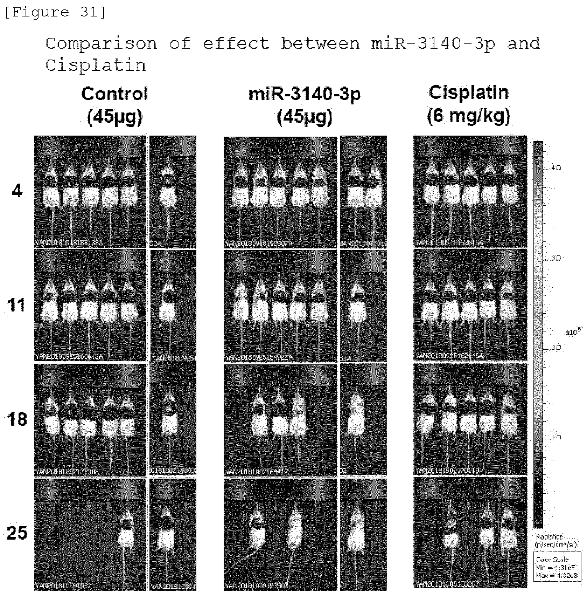

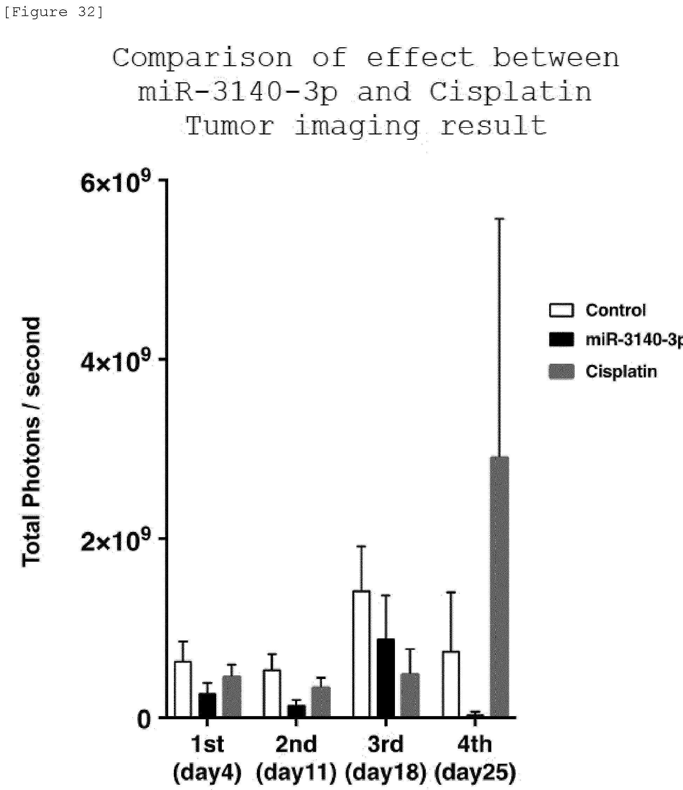

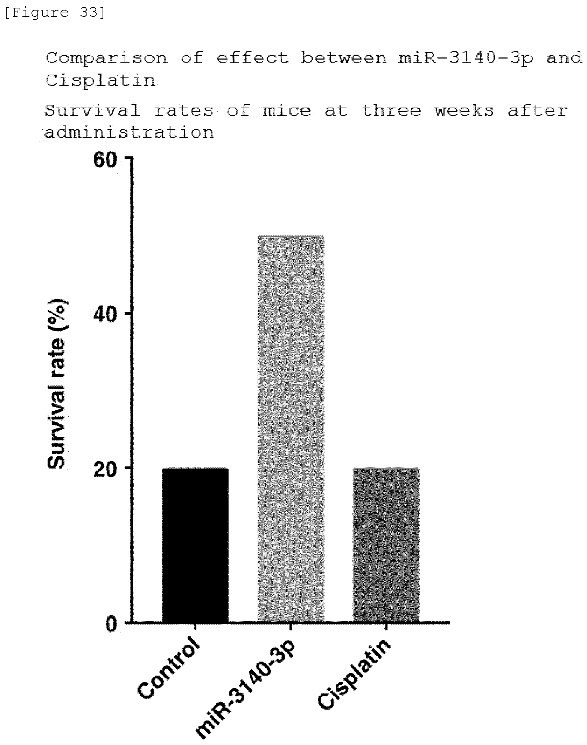

### Experiment 9. Comparison of antitumor effect with chemotherapy agents (Figures 30-33)

In order to investigate the relative superiority of antitumor effect of miR-3140-3p, the tumor suppression effect of cisplatin which is the first-line drug of malignant pleural mesothelioma and the tumor suppression effect of miR-3140-3p were compared *in vivo.*

Six weeks-old male mice (C-B-17/Icr-scid/scid Jcl) were used as mice. Malignant pleural mesothelioma cell strain EHMES-10 which expresses the luciferase gene was used as the tumor cell.

The protocol is shown below.
1: Mice were intraperitoneally administered 0.1 mL per 10 g of body weight of a mixed anesthetic drug of medetomidine hydrochloride, midazolam, and butorphanol tartrate.
2: After anesthesia, mouse chest hair was shaved, and an incision was made in the epidermis with scissors.
3: In the mouse pleural cavity 100 µL of tumor cells (3 x 10⁷ cells/mL) was transplanted with a 27 G syringe for insulin.
4: Four days after transplantation, imaging of tumor cells was performed with IVIS Spectrum CT *In vivo* Imaging System.
5: After imaging, grouping was performed with successfully transplanted mice.
6: Mice were intraperitoneally administered 0.1 mL per 10 g of body weight of a mixed anesthetic drug of medetomidine hydrochloride, midazolam, and butorphanol tartrate to anesthetize the mice.
7: After anesthesia, 100 µL of the miRNA/A6K mixture was administered in the pleural cavity for the miRNA administration group, and cisplatin (6mg/kg) was intraperitoneally administered to cisplatin administration group.
8: Imaging was performed every week from the first imaging, and tumor expansion was observed.

The mixed anesthetic drug was prepared as in Table 4 shown above. The miRNA/A6K mixture was prepared as in Tables 5 and 6 shown above. Cisplatin was prepared as in Table 7 below.

**[Table 11]**

| Table 7 Preparation of cisplatin | |
|---|---|
| | /mL |
| Cisplatin (25mg/50mL) | 600 *µ* L |
| DW | 400 *µ* L |

Note that the same control RNA as that used in Experiment 7 was employed as the control.

Experimental results are shown in Figures 30-33. As shown in each figure, it was shown that miR-3140-3p may exert an antitumor effect that is equivalent or higher compared to cisplatin which is also the first-line drug of malignant pleural mesothelioma.

## Claims

1. A pharmaceutical composition for use in cancer therapy comprising an RNA which functions as a mature miRNA, **characterized in that** said RNA comprises:
(i) a mature-miRNA consisting of a sequence represented by SEQ ID NO. 11;
(ii) an RNA having a substitution, addition, and/or deletion of 1 - 5 bases to a mature-miRNA consisting of the sequence represented by SEQ ID NO. 11, and having cancer therapeutic effect; or
(iii) an RNA having 80% or more sequence identity to a mature-miRNA consisting of the sequence represented by SEQ ID NO. 11, and having a cancer therapeutic effect.

2. The pharmaceutical composition for the use according to claim 1, **characterized in that** said cancer is a solid cancer.

3. The pharmaceutical composition for the use according to claim 2, **characterized in that** said solid cancer is colon cancer, pancreatic cancer, tongue cancer, mesothelioma, uterine sarcoma, osteosarcoma, breast cancer, lung cancer, or head and neck cancer.

4. The pharmaceutical composition for the use according to any one of claims 1 to 3, **characterized in that** said miRNA is chemically modified.

5. The pharmaceutical composition for the use according to claim 4, **characterized in that** said chemical modification is one or more chemical modifications selected from the group consisting of LNA-tion, BNA-tion, ENA-ation, 2'-OMe modification, phosphorothioation, S-TuD-ation, morpholino modification, peptide addition, glycosylation, aptamer addition, hydrophobic molecule addition, polymer addition, and addition of unmodified DNA.

6. The pharmaceutical composition for the use according to any one of claims 1 to 5, **characterized in that** it is used in combination with a nucleic acid transfection agent.

7. The pharmaceutical composition for the use according to claim 6, **characterized in that** said transfection agent is a lipid-based transfection agent, a polymer-based transfection agent, a magnetic particle-based transfection agent, an exosome for nucleic acid delivery, or a viral protein for nucleic acid delivery.

8. The pharmaceutical composition for the use according to claim 6, **characterized in that** said transfection agent is a transfection agent comprising a peptide represented by amino acid sequences GGGGDD (G4D2), GGGGGGDD (G6D2), GGGGGGGGDD (G8D2), GGGGGGGGGGDD (G10D2), AAAAAAD (A6D), AAAAAADD (A6D2), AAAAAAK (A6K), AAAAAAKK (A6K2), VVVVVVD (V6D), VVVVVVDD (V6D2), VVVVVVK (V6K), VVVVVVKK (V6K2), LLLLLLD (L6D), LLLLLLDD (L6D2), LLLLLLK (L6K), or LLLLLLKK (L6K2).

9. The pharmaceutical composition for the use according to any one of claims 1 to 8, **characterized in that** it is administered via topical administration.

10. The pharmaceutical composition for the use according to any one of claims 1 to 9, **characterized in that** it is used in combination with other anticancer agents.

11. The pharmaceutical composition for the use according to claim 10, **characterized in that** said other anticancer agents are one or more anticancer agents selected from the group consisting of an alkylating agent, a platinum preparation, a metabolism antagonist, a topoisomerase inhibitor, a microtubular inhibitor, an anti-cancerous antibiotic, a molecular target drug, a hormone preparation, an immunomodulation drug, an interferon, an interleukin, and a plant-derived anticancer agent.

## Patentansprüche

1. Pharmazeutische Zusammensetzung zur Verwendung in der Krebstherapie, umfassend eine RNA, die als reife miRNA fungiert, **dadurch gekennzeichnet, dass** die RNA umfasst:
(i) eine reife miRNA, die aus einer durch SEQ ID NO. 11 dargestellten Sequenz besteht;
(ii) eine RNA, die eine Substitution, Addition und/oder Deletion von 1-5 Basen gegenüber einer reifen miRNA aufweist, die aus der durch SEQ ID NO. 11 dargestellten Sequenz besteht, und die eine krebstherapeutische Wirkung besitzt; oder
(iii) eine RNA, die eine Sequenzidentität von 80 % oder mehr gegenüber einer reifen miRNA aufweist, die aus der durch SEQ ID NO. 11 dargestellten Sequenz besteht, und die eine krebstherapeutische Wirkung besitzt.

2. Pharmazeutische Zusammensetzung zur Verwendung nach Anspruch 1, **dadurch gekennzeichnet, dass** es sich bei dem Krebs um einen soliden Krebs handelt.

3. Pharmazeutische Zusammensetzung zur Verwendung nach Anspruch 2, **dadurch gekennzeichnet, dass** es sich bei dem soliden Krebs um Dickdarmkrebs, Bauchspeicheldrüsenkrebs, Zungenkrebs, Mesotheliom, Uterussarkom, Osteosarkom, Brustkrebs, Lungenkrebs oder Kopf-Hals-Krebs handelt.

4. Pharmazeutische Zusammensetzung zur Verwendung nach einem der Ansprüche 1 bis 3, **dadurch gekennzeichnet, dass** die miRNA chemisch modifiziert ist.

5. Pharmazeutische Zusammensetzung zur Verwendung nach Anspruch 4, **dadurch gekennzeichnet, dass** die chemische Modifikation eine oder mehrere chemische Modifikationen ist, ausgewählt aus der Gruppe bestehend aus LNA-ierung, BNA-ierung, ENA-ierung, 2'-OMe-Modifikation, Phosphorthioatisierung, S-TuD-ierung, Morpholino-Modifikation, Peptid-Addition, Glykosylierung, Aptamer-Addition, Addition eines hydrophoben Moleküls, Polymer-Addition und Addition von unmodifizierter DNA.

6. Pharmazeutische Zusammensetzung zur Verwendung nach einem der Ansprüche 1 bis 5, **dadurch gekennzeichnet, dass** sie in Kombination mit einem Nukleinsäure-Transfektionsmittel verwendet wird.

7. Pharmazeutische Zusammensetzung zur Verwendung nach Anspruch 6, **dadurch gekennzeichnet, dass** das Transfektionsmittel ein Transfektionsmittel auf Lipidbasis, ein Transfektionsmittel auf Polymerbasis, ein Transfektionsmittel auf Basis magnetischer Partikel, ein Exosom zur Nukleinsäurezufuhr oder ein virales Protein zur Nukleinsäurezufuhr ist.

8. Pharmazeutische Zusammensetzung zur Verwendung nach Anspruch 6, **dadurch gekennzeichnet, dass** das Transfektionsmittel ein Transfektionsmittel ist, das ein Peptid umfasst, das durch die Aminosäuresequenzen GGGGDD (G4D2), GGGGGGDD (G6D2), GGGGGGGGDD (G8D2), GGGGGGGGGGDD (G10D2), AAAAAAD (A6D), AAAAAADD (A6D2), AAAAAAK (A6K), AAAAAAKK (A6K2), VVVVVVD (V6D), VVVVVVDD (V6D2), VVVVVVK (V6K), VVVVVVKK (V6K2), LLLLLLD (L6D), LLLLLLDD (L6D2), LLLLLLK (L6K), oder LLLLLLKK (L6K2) dargestellt wird.

9. Pharmazeutische Zusammensetzung zur Verwendung nach einem der Ansprüche 1 bis 8, **dadurch gekennzeichnet, dass** sie topisch verabreicht wird.

10. Pharmazeutische Zusammensetzung zur Verwendung nach einem der Ansprüche 1 bis 9, **dadurch gekennzeichnet, dass** sie in Kombination mit anderen Antikrebsmitteln verwendet wird.

11. Pharmazeutische Zusammensetzung zur Verwendung nach Anspruch 10, **dadurch gekennzeichnet, dass** die anderen Antikrebsmittel ein oder mehrere Antikrebsmittel sind, ausgewählt aus der Gruppe bestehend aus einem Alkylierungsmittel, einem Platinpräparat, einem Stoffwechselantagonisten, einem Topoisomerase-Inhibitor, einem Mikrotubuli-Inhibitor, einem krebsbekämpfenden Antibiotikum, einem molekular zielgerichteten Arzneimittel, einem Hormonpräparat, einem immunmodulierenden Arzneimittel, einem Interferon, einem Interleukin und einem pflanzlichen Antikrebsmittel.

## Revendications

1. Composition pharmaceutique pour son utilisation dans une thérapie contre le cancer comprenant un ARN qui fonctionne comme un miARN mature, **caractérisée en ce que** ledit ARN comprend :
(i) un miARN mature constitué d'une séquence représentée par SEQ ID NO. 11 ;
(ii) un ARN ayant une substitution, un ajout, et/ou une délétion de 1 à 5 bases à un miARN mature constitué de la séquence représentée par SEQ ID NO. 11, et ayant un effet thérapeutique contre le cancer ; ou
(iii) un ARN ayant une identité de séquence de 80 % ou plus avec un miARN mature constitué de la séquence représentée par SEQ ID NO. 11, et ayant un effet thérapeutique contre le cancer.

2. Composition pharmaceutique pour son utilisation selon la revendication 1, **caractérisée en ce que** ledit cancer est un cancer solide.

3. Composition pharmaceutique pour son utilisation selon la revendication 2, **caractérisée en ce que** ledit cancer solide est un cancer du côlon, un cancer du pancréas, un cancer de la langue, un mésothéliome, un sarcome utérin, un ostéosarcome, un cancer du sein, un cancer du poumon, ou un cancer de la tête et du cou.

4. Composition pharmaceutique pour son utilisation selon l'une quelconque des revendications 1 à 3, **caractérisée en ce que** ledit miARN est chimiquement modifié.

5. Composition pharmaceutique pour son utilisation selon la revendication 4, **caractérisée en ce que** ladite modification chimique est une ou plusieurs modifications chimiques sélectionnées dans le groupe constitué des LNA-tion, BNA-tion, ENA-ation, modification 2'-OMe, phosphorothioation, S-TuD-ation, modification morphilino, ajout de peptide, glycosylation, ajout d'aptamère, ajout de molécule hydrophobe, ajout de polymère, et ajout d'ADN non modifié.

6. Composition pharmaceutique pour son utilisation selon l'une quelconque des revendications 1 à 5, **caractérisée en ce qu'**elle est utilisée en combinaison avec un agent de transfection d'acide nucléique.

7. Composition pharmaceutique pour son utilisation selon la revendication 6, **caractérisée en ce que** ledit agent de transfection est un agent de transfection à base de lipide, un agent de transfection à base de polymère, un agent de transfection à base de particules magnétiques, un exosome pour la délivrance d'acide nucléique, ou une protéine virale pour la délivrance d'acide nucléique.

8. Composition pharmaceutique pour son utilisation selon la revendication 6, **caractérisée en ce que** ledit agent de transfection est un agent de transfection comprenant un peptide représenté par les séquences d'acides aminés GGGGDD (G4D2), GGGGGGDD (G6D2), GGGGGGGGDD (G8D2), GGGGGGGGGGDD (G10D2), AAAAAAD (A6D), AAAAAADD (A6D2), AAAAAAK (A6K), AAAAAAKK (A6K2), VVVVVVD (V6D), VVVVVVDD (V6D2), VVVVVVK (V6K), VVVVVVKK (V6K2), LLLLLLD (L6D), LLLLLLDD (L6D2), LLLLLLK (L6K), ou LLLLLLKK (L6K2).

9. Composition pharmaceutique pour son utilisation selon l'une quelconque des revendications 1 à 8, **caractérisée en ce qu'**elle est administrée par administration topique.

10. Composition pharmaceutique pour son utilisation selon l'une quelconque des revendications 1 à 9, **caractérisée en ce qu'**elle est utilisée en combinaison avec d'autres agents anticancéreux.

11. Composition pharmaceutique pour son utilisation selon la revendication 10, **caractérisée en ce que** lesdits autres agents anticancéreux sont un ou plusieurs agents anticancéreux sélectionnés dans le groupe constitué d'un agent d'alkylation, d'une préparation de platine, d'un antagoniste du métabolisme, d'un inhibiteur de la topoisomérase, d'un inhibiteur microtubulaire, d'un antibiotique anti-cancéreux, d'un médicament cible moléculaire, d'une préparation hormonale, d'un médicament immunomodulateur, d'un interféron, d'une interleukine, et d'un agent anticancéreux d'origine végétale.
